(19) Europäisches Patentamt / European Patent Office / Office européen des brevets

(11) **EP 4 502 591 A1**

(12) **EUROPEAN PATENT APPLICATION**
published in accordance with Art. 153(4) EPC

(43) Date of publication:
**05.02.2025 Bulletin 2025/06**

(21) Application number: **23780115.4**

(22) Date of filing: **24.03.2023**

(51) International Patent Classification (IPC):
*G01N 27/416* (2006.01)

(52) Cooperative Patent Classification (CPC):
**G01N 27/416**

(86) International application number:
**PCT/JP2023/011705**

(87) International publication number:
**WO 2023/190105 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.03.2022 JP 2022059560**

(71) Applicant: **TOKUYAMA CORPORATION
Yamaguchi 745-8648 (JP)**

(72) Inventors:
• **UMEMOTO, Shiori
  Shunan-shi, Yamaguchi 745-8648 (JP)**
• **KIKUCHI, Shigetoshi
  Shunan-shi, Yamaguchi 745-8648 (JP)**
• **WATANABE, Shin
  Shunan-shi, Yamaguchi 745-8648 (JP)**

(74) Representative: **Hoffmann Eitle
Patent- und Rechtsanwälte PartmbB
Arabellastraße 30
81925 München (DE)**

(54) **ION CONCENTRATION MEASUREMENT DEVICE AND MEASUREMENT METHOD**

(57) An object of the present invention is to provide an ion concentration measurement device and a measurement method capable of precisely determining an ion concentration by clarifying a method for selecting an ion-selective electrode that has a small measurement error and hardly receives interference of a coexisting ion and using the selected ion-selective electrode. An ion concentration measurement device including two or more kinds of ion-selective electrodes that have mutually different selectivity coefficient ratios for a plurality of ions and satisfy formulas (1) and (2), wherein the two or more kinds of ion-selective electrodes are constituted by n kinds of ion-selective electrodes, and wherein n is the number of kinds of the plurality of ions and is an integer of 2 or more.

$$\text{Matrix A} = \begin{pmatrix} K_{S1}(B1,B1) & K_{S1}(B1,B2) & \cdots & K_{S1}(B1,Bj) & \cdots & K_{S1}(B1,Bn) \\ K_{S2}(B1,B1) & K_{S2}(B1,B2) & \cdots & K_{S2}(B1,Bj) & \cdots & K_{S2}(B1,Bn) \\ \vdots & \vdots & & \vdots & & \vdots \\ K_{Si}(B1,B1) & K_{Si}(B1,B2) & \cdots & K_{Si}(B1,Bj) & \cdots & K_{Si}(B1,Bn) \\ \vdots & \vdots & & \vdots & & \vdots \\ K_{Sn}(B1,B1) & K_{Sn}(B1,B2) & \cdots & K_{Sn}(B1,Bj) & \cdots & K_{Sn}(B1,Bn) \end{pmatrix} \quad (1)$$

$$\|A\| > 0.1 \quad (2)$$

In formula (1), Bj represents a j-th ion, and Si is a symbol representing an i-th ion-selective electrode. $K_{Si}(B1, Bj)$ represents an ion selectivity coefficient for the ion Bj when an ion B1 is used as a reference in the ion-selective electrode Si. In formula (2), $\|A\|$ is an absolute value of a determinant of a matrix A.

[Fig. 1]

## Description

Technical Field

The present invention relates to an ion concentration measurement device and a measurement method.

Background Art

An ion-selective electrode is generally used for measurement of an ion concentration in blood in a clinical examination. By immersing the ion-selective electrode and a reference electrode in a sample solution, a potential difference corresponding to an ion concentration of the sample solution is obtained. Therefore, by grasping a relationship between the potential difference and the concentration in advance, the ion concentration in the sample solution can be found from the potential difference. Since the ion concentration in the sample solution can be measured simply and quickly only by immersing the electrodes in the sample solution, ion concentration measurement using the ion-selective electrode is widely used currently.

A case where one kind of ion concentration is measured using an ion-selective electrode S and a reference electrode will be briefly described. A relationship between a potential difference Es obtained when the ion-selective electrode S and the reference electrode are immersed in a sample solution containing an ion $X^a$ and an ion concentration $(C(X^a))$ in the sample solution can be expressed by a Nernst equation (formula (4)).

[Mathematical formula 1]

$$E_S = E_{0,S} + \frac{2.303RT}{aF} \times \log_{10} C(X^a) \qquad (4)$$

In the formula (4),

$E_S$ is a potential difference generated between the ion-selective electrode S and the reference electrode,
$E_{0,S}$ is a standard potential difference generated between the ion-selective electrode S and the reference electrode,
R is a gas constant,
T is an absolute temperature,
a is a valence of the ion,
F is a Faraday constant, and
$C(X^a)$ is a concentration of the ion $X^a$.

By measuring a solution containing the ion $X^a$ having a known concentration with the ion-selective electrode S, a calibration curve representing formula (4) can be created. Using the created calibration curve, an unknown sample solution is measured, and an ion concentration can be calculated from a potential difference obtained.

Note that the sample solution may contain, in addition to an ion to be measured (hereinafter, referred to as "measurement ion"), an ion other than the measurement ion (hereinafter, referred to as "coexisting ion"). Here, when a concentration of a measurement ion B1 contained in a sample solution containing n kinds of ions consisting of the measurement ion B1 and coexisting ions B2 to Bn is measured using the ion-selective electrode S and the reference electrode, there is a relationship of a Nicolsky-Eisenmann equation (formula (5)).

[Mathematical formula 2]

$$E_S = E_{0,S} + \frac{2.303RT}{aF} \times \log_{10}[C(B1) + \sum_{j=2}^{n} K_S(B1,Bj) \, C(Bj)] \qquad (5)$$

In the formula (5),

is a potential difference generated between the ion-selective electrode S and the reference electrode,
$E_{0,S}$ is a standard potential difference generated between the ion-selective electrode S and the reference electrode,
R is a gas constant,
T is an absolute temperature,
a is a valence of the ion B1,
F is a Faraday constant,

Bj represents a j-th ion among the n kinds of ions,
j is an integer of 2 to n, and
C(Bj) is a concentration of the ion Bj.

Here, $K_S(B1, Bj)$ is an ion selectivity coefficient of the ion-selective electrode S for the ion Bj when the ion B1 is used as a reference in the ion-selective electrode S. In other words, $K_S(B1, Bj)$ is a coefficient representing a magnitude of an influence of the ion Bj on a potential difference generated between the ion-selective electrode S and the reference electrode by a ratio when the ion B1 is used as a reference.

When the above measurement is performed on a sample solution containing the ion B1 having a concentration C(B1) but not containing the coexisting ions B2 to Bn using an ion-selective electrode S having an ion selectivity coefficient for the ion B2 when the ion B1 is used as a reference is Ks(B1, B2), the following potential difference $E_{B1}$ is obtained.
[Mathematical formula 3]

$$E_{B1} = E_{0,S} + \frac{2.303RT}{aF} \times \log_{10} C(B1) \qquad (6)$$

Furthermore, when the ion B2 having a concentration C(B2) is added to this sample solution (that is, in a case of a sample solution containing the ion B1 having the concentration C(B1) and the ion B2 having the concentration C(B2) but not containing the coexisting ions B3 to Bn), and the above measurement is performed, the following potential difference $E_{B1+B2}$ is obtained.
[Mathematical formula 4]

$$E_{B1+B2} = E_{0,S} + \frac{2.303RT}{aF} \times \log_{10}[C(B1) + K_S(B1,B2) C(B2)] \qquad (7)$$

Here, in the above formulas (6) and (7), $E_{0,S}$, R, T, a, and F are the same as those defined in the above formula (5), respectively, and $E_{B1}$ in the above formula (6) and $E_{B1+B2}$ in the above formula (7) correspond to in the above formula (5).

As suggested from the above formulas (6) and (7), when the ion selectivity coefficient Ks(B1, B2) is large, B2 ion interference occurs when the ion B1 is measured, which causes an error.

Therefore, in order to measure a concentration of a specific ion, a selectivity coefficient for another ion is desirably as small as possible. It is known that, when an ion-selective membrane containing bis[(12-crown-4) methyl] 2-dodecyl-2-methylmalonate [CAS No. 80403-59-4] is used for a sodium ion-selective electrode, a selectivity coefficient $K_S(Na^+, K^+)$ of a potassium ion to a sodium ion is 0.01, which is put to practical use (Non Patent Document 1).

However, depending on an ion to be measured, it may be difficult to develop an electrode having a sufficiently small ion selectivity coefficient for another ion. In particular, there are few ion-selective membranes suitable for measurement of an anion, and an ion-exchange membrane that is not sufficiently selective for an ion to be measured is often used for the measurement of an anion. For example, in order to measure a bicarbonate ion ($HCO_3^-$) in blood, an electrode satisfying a selectivity coefficient $K_S(HCO_3^-, Cl^-)$ of 0.01 or less is required, but only an electrode having $K_S(HCO_3^-, Cl^-)$ of more than 1 is known. In a clinical examination, presence of an ion that responds more strongly than an ion to be measured in a sample solution is a problem in practical use of an electrode that measures a bicarbonate ion ($HCO_3^-$) concentration in blood.

Therefore, a method for measuring a plurality of kinds of ion concentrations using a plurality of ion-selective electrodes having insufficient selectivity for a measurement ion has been developed. Examples thereof include Patent Documents 1, 2, and 3. There is also an example using an ion-selective electrode for removing an influence of an interfering ion in addition to an ion-selective electrode used for measurement of an ion concentration (Patent Document 4).

Citation List

Patent Documents

Patent Document 1: JP H1-502360 A
Patent Document 2: JP S54-030094 A
Patent Document 3: WO 2019/163281 A
Patent Document 4: JP H07-167818 A

Non Patent Document

Non Patent Document 1: Anal. Chem., 1982, 54(7), 1224-1227

Summary of Invention

Technical Problem

As in Patent Document 2, a method is known in which simultaneous equations are solved for values calculated from n measured values obtained with a plurality of (n) ion-selective electrodes having low ion selectivity, to calculate concentrations of n kinds of ions present in a sample solution. However, there is an error in a measured value in an actual measurement. In addition, when the sample solution contains n+1 or more kinds of ions, interference of a coexisting ion cannot be ignored. Therefore, as a result of studies by the present inventors, it has become clear that depending on selection of an ion-selective electrode, an ion concentration cannot be precisely determined due to a measurement error or interference of a coexisting ion.

Therefore, an object of the present invention is to provide an ion concentration measurement device and a measurement method capable of precisely determining an ion concentration by clarifying a method for selecting an ion-selective electrode that has a small measurement error and hardly receives interference of a coexisting ion and using the selected ion-selective electrode.

Solution to Problem

The present inventors have conducted intensive studies in order to solve the above problems. As a result, the present inventors have found an ion concentration measurement device and a measurement method capable of precisely determining an ion concentration by clarifying a method for selecting an ion-selective electrode that has a small measurement error and hardly receives interference of a coexisting ion and using the selected ion-selective electrode.

That is, a first aspect of the present invention provides an ion concentration measurement device including two or more kinds of ion-selective electrodes that have mutually different selectivity coefficient ratios for a plurality of ions and satisfy the following formulas (1) and (2), wherein the two or more kinds of ion-selective electrodes are constituted by n kinds of ion-selective electrodes, and wherein n is the number of kinds of the plurality of ions and is an integer of 2 or more.

[Mathematical formula 5]

$$\text{Matrix A} = \begin{pmatrix} K_{S1}(B1,B1) & K_{S1}(B1,B2) & \cdots & K_{S1}(B1,Bj) & \cdots & K_{S1}(B1,Bn) \\ K_{S2}(B1,B1) & K_{S2}(B1,B2) & \cdots & K_{S2}(B1,Bj) & \cdots & K_{S2}(B1,Bn) \\ \vdots & \vdots & & \vdots & & \vdots \\ K_{Si}(B1,B1) & K_{Si}(B1,B2) & \cdots & K_{Si}(B1,Bj) & \cdots & K_{Si}(B1,Bn) \\ \vdots & \vdots & & \vdots & & \vdots \\ K_{Sn}(B1,B1) & K_{Sn}(B1,B2) & \cdots & K_{Sn}(B1,Bj) & \cdots & K_{Sn}(B1,Bn) \end{pmatrix} \quad (1)$$

$$\|A\| > 0.1 \quad (2)$$

In formula (1), each component of the matrix A represents an ion selectivity coefficient, wherein Bj represents a j-th ion among n kinds of ions constituting the plurality of ions,

Si is a symbol representing an i-th ion-selective electrode constituting the n kinds of ion-selective electrodes,
i and j are each independently an integer of 1 to n, and
$K_{Si}(B1, Bj)$ represents an ion selectivity coefficient for the ion Bj when an ion B1 is used as a reference in the ion-selective electrode Si.

In formula (2), $\|A\|$ is an absolute value of a determinant of the matrix A.

In addition, a second aspect of the present invention provides a measurement method including a step of measuring an ion concentration in a sample solution using a plurality of ion-selective electrodes satisfying the above formulas (1) and (2).

Advantageous Effects of Invention

The present invention has clarified a method for selecting an ion-selective electrode that has a small measurement error and hardly receives interference of a coexisting ion even when the number of kinds (n+1 or more) of coexisting ions is more than the number of electrodes (n). By selecting an ion-selective electrode having a large absolute value ($\|A\|$) of the determinant of the matrix A, it is possible to precisely measure an ion concentration with a small relative standard deviation of a measurement result. The present invention can provide an ion concentration measurement device and a measurement method capable of precisely determining an ion concentration.

Brief Description of Drawings

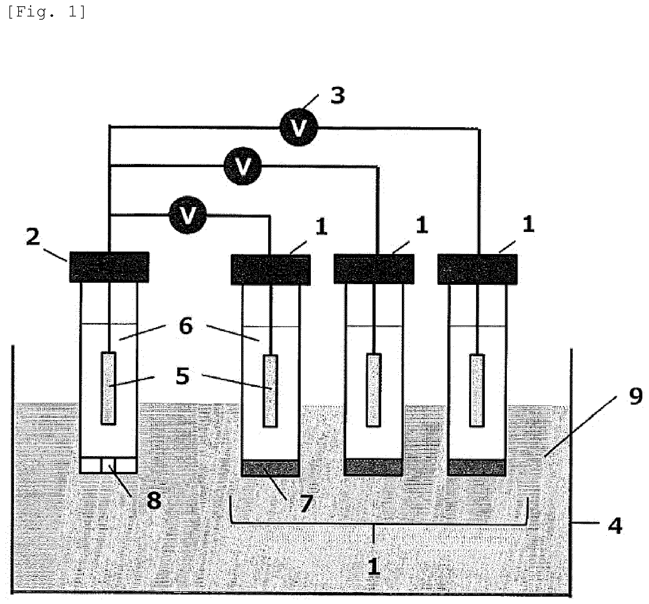

Fig. 1 is a schematic diagram illustrating an ion concentration measurement device according to an embodiment of the present invention.

Description of Embodiments

Hereinafter, an embodiment for carrying out the present invention will be described.

[Description of formulas (1), (2), and (3)]

First, formulas (1), (2), and (3) used in the present invention will be described. By using formulas (1) and (2), it is possible to select an ion-selective electrode that has a small measurement error and is resistant to interference of a coexisting ion. As a result, it is possible to provide an ion concentration measurement device capable of precisely determining an ion concentration, which is a characteristic of the present invention.

As a preceding stage for describing a case of measurement with n kinds of ion-selective electrodes, a case of measurement with one kind of ion-selective electrode will be described.

<u>Case of measurement with one kind of ion-selective electrode</u>

When a concentration of a specific ion (ion B1) contained in a sample solution containing n kinds (n is an integer of 2 or more) of ions is measured using one kind of ion-selective electrode S (hereinafter, also referred to as "electrode S") and a reference electrode, there is a relationship of a Nicolsky-Eisenmann equation (formula (5)). Here, (2.303RT)/aF is referred to as Slope. That is, Slope is a constant. In practice, Slope often does not present a theoretical value for various reasons such as electrical resistance in a device and a liquid junction potential. In this case, it is preferable to determine Slope by creating a calibration curve.

[Mathematical formula 6]

$$E_S = E_{0,S} + \frac{2.303RT}{aF} \times \log_{10}[C(B1) + \sum_{j=2}^{n} K_S(B1,Bj)\, C(Bj)] \quad (5)$$

$$= E_{0,S} + \text{Slope} \times \log_{10}[C(B1) + \sum_{j=2}^{n} K_S(B1,Bj)\, C(Bj)] \quad (5')$$

wherein

is a potential difference generated between the ion-selective electrode S and the reference electrode,
$E_{0,S}$ is a standard potential difference generated between the ion-selective electrode S and the reference electrode,
Slope is 2.303RT/aF,
R is a gas constant,
T is an absolute temperature,
a is a valence of the ion B1,
F is a Faraday constant,
Bj represents a j-th ion among n kinds of ions,
j is an integer of 2 to n,

C(Bj) is a concentration of the ion Bj, and

$K_S(B1, Bj)$ is a selectivity coefficient for the ion Bj when an ion B1 is used as a reference in the electrode S.

A sample solution (calibration curve solution) having a known concentration of the ion B1 is measured using the ion-selective electrode S and the reference electrode, and a calibration curve representing formula (5') can be created. From the calibration curve, Slope and a standard potential difference $E_{0,S}$ at the time of measurement with the ion-selective electrode S can be determined.

Formula (5') is transferred, and the following formula (8) can be derived. Using this formula (8), from $E_{0,S}$ and a potential difference obtained when the ion-selective electrode S and the reference electrode are immersed in the sample solution, an ion concentration when n kinds of ion concentrations in the sample solution are converted into any ion B1 concentration can be calculated.

[Mathematical formula 7]

$$C(B1) + \sum_{j=2}^{n} K_S(B1,Bj)\, C(Bj) = 10^{\left( \frac{E_S - E_{0,S}}{Slope} \right)} \tag{8}$$

Here, when n = 2, the above formula (8) can be expressed as the following formula (8A).

[Mathematical formula 8]

$$C(B1) + K_S(B1,B2)\, C(B2) = 10^{\left( \frac{E_S - E_{0,S}}{Slope} \right)} \tag{8A}$$

Then, the above formula (8A) is transferred, and can be further transformed into the following formula (8B).

[Mathematical formula 9]

$$K_S(B1,B2) = \frac{10^{\left( \frac{E_S - E_{0,S}}{Slope} \right)} - C(B1)}{C(B2)} \tag{8B}$$

That is, when n = 2, $K_S(B1, B2)$, which is a selectivity coefficient for the ion B2 when the ion B1 is used as a reference in the electrode S, can be determined using the above formula (8B).

Case of measurement with n kinds of ion-selective electrodes (description of formula (1))

A case where n kinds of ions (ions: B1, B2, ···, Bj, ···, and Bn, ion concentrations: C(B1), C(B2), ···, C(Bj), ···, and C(Bn)) in a sample solution are measured using n kinds (n is an integer of 2 or more) of ion-selective electrodes S1 to Sn will be described. At this time, the above formula (8) is established for each of the n kinds of ion-selective electrodes S1 to Sn. Therefore, simultaneous equations of formula (9) are established.

[Mathematical formula 10]

$$C(B1)+K_{S1}(B1,B2)C(B2) \\ + \cdots + K_{S1}(B1,Bj)C(Bj) \\ + \cdots + K_{S1}(B1,Bn)C(Bn) = 10^{\left(\dfrac{E_{S1} - E_{0,S1}}{Slope}\right)}$$

$$C(B1)+K_{S2}(B1,B2)C(B2) \\ + \cdots + K_{S2}(B1,Bj)C(Bj) \\ + \cdots + K_{S2}(B1,Bn)C(Bn) = 10^{\left(\dfrac{E_{S2} - E_{0,S2}}{Slope}\right)} \qquad (9)$$

$$\vdots \qquad\qquad \vdots$$

$$C(B1)+K_{Sn}(B1,B2)C(B2) \\ + \cdots + K_{Sn}(B1,Bj)C(Bj) \\ + \cdots + K_{Sn}(B1,Bn)C(Bn) = 10^{\left(\dfrac{E_{Sn} - E_{0,Sn}}{Slope}\right)}$$

$$\text{Calculated value obtained with electrode } Si \ (i = n)$$

$$\text{Calculated value obtained with electrode } Si \ (i \text{ is an integer of 1 to n}) = 10^{\left(\dfrac{E_{Si} - E_{0,Si}}{Slope}\right)}$$

In formula (9),

Bj represents a j-th ion among the n kinds of ions,
Si is a symbol representing an i-th ion-selective electrode constituting the n kinds of ion-selective electrodes,
i and j are each independently an integer of 1 to n,
$K_{Si}(B1, Bj)$ is Bj when an ion-selective electrode Si (i-th ion-selective electrode) is used as the ion-selective electrode S, that is, an ion selectivity coefficient for the ion Bj when an ion B1 is used as a reference in the ion-selective electrode Si,
$E_{Si}$ is a potential difference generated between the ion-selective electrode Si and a reference electrode,
$E_{0,Si}$ is a standard potential difference generated between the ion-selective electrode Si and the reference electrode,
Slope is 2.303RT/aF, and
R, T, a, and F are the same as R, T, a, and F defined by the above formula (5), respectively. Here, the right side of formula (9) is a calculated value obtained with the ion-selective electrode Si. This value is a value calculated using the potential difference $E_{Si}$, the Slope determined by the calibration curve, and the standard potential differences $E_{0,Si}$.

When the simultaneous equations of the above formula (9) are converted into a form of a matrix, the following formula (10) is obtained.
[Mathematical formula 11]

$$\begin{pmatrix} 1 & K_{S1}(B1,B2) & \cdots & K_{S1}(B1,Bj) & \cdots & K_{S1}(B1,Bn) \\ 1 & K_{S2}(B1,B2) & \cdots & K_{S2}(B1,Bj) & \cdots & K_{S2}(B1,Bn) \\ \vdots & \vdots & & \vdots & & \vdots \\ 1 & K_{Sn}(B1,B2) & \cdots & K_{Sn}(B1,Bj) & \cdots & K_{Sn}(B1,Bn) \end{pmatrix} \begin{pmatrix} C(B1) \\ C(B2) \\ \vdots \\ C(Bn) \end{pmatrix} = \begin{pmatrix} \text{Calculated value obtained with electrode S1} \\ \text{Calculated value obtained with electrode S2} \\ \vdots \\ \text{Calculated value obtained with electrode Sn} \end{pmatrix} \quad (10)$$

Matrix A'

A matrix on the left side of formula (9) is referred to as a matrix A'. Here, for each of the n kinds of ion-selective electrodes S1, S2, $\cdots$, and Sn, a selectivity coefficient $K_S(B1,B1)$ (that is, $K_{S1}(B1, B1)$, $K_{S2}(B1, B1)$, $\cdots$, and $K_{Sn}(B1, B1)$) for any ion B1 itself serving as a reference is 1. Therefore, when 1 is rewritten as $K_S(B1, B1)$ (that is, $K_{S1}(B1, B1)$, $K_{S2}(B1, B1)$, $\cdots$, and $K_{Sn}(B1, B1)$ for n kinds of ion-selective electrodes S1, S2, $\cdots$, and Sn, respectively), the matrix A' can be transformed as in formula (11). The right side of formula (11) corresponds to the matrix A (formula (1)) of the present invention.

[Mathematical formula 12]

$$\begin{pmatrix} 1 & K_{S1}(B1,B2) & \cdots & K_{S1}(B1,Bj) & \cdots & K_{S1}(B1,Bn) \\ 1 & K_{S2}(B1,B2) & \cdots & K_{S2}(B1,Bj) & \cdots & K_{S2}(B1,Bn) \\ \vdots & \vdots & & \vdots & & \vdots \\ 1 & K_{Sn}(B1,B2) & \cdots & K_{Sn}(B1,Bj) & \cdots & K_{Sn}(B1,Bn) \end{pmatrix}$$

Matrix A'

$$= \begin{pmatrix} K_{S1}(B1,B1) & K_{S1}(B1,B2) & \cdots & K_{S1}(B1,Bj) & \cdots & K_{S1}(B1,Bn) \\ K_{S2}(B1,B1) & K_{S2}(B1,B2) & \cdots & K_{S2}(B1,Bj) & \cdots & K_{S2}(B1,Bn) \\ \vdots & \vdots & & \vdots & & \vdots \\ K_{Sn}(B1,B1) & K_{Sn}(B1,B2) & \cdots & K_{Sn}(B1,Bj) & \cdots & K_{Sn}(B1,Bn) \end{pmatrix} \quad (11)$$

Matrix A

$$\text{Matrix A} = \begin{pmatrix} K_{S1}(B1,B1) & K_{S1}(B1,B2) & \cdots & K_{S1}(B1,Bj) & \cdots & K_{S1}(B1,Bn) \\ K_{S2}(B1,B1) & K_{S2}(B1,B2) & \cdots & K_{S2}(B1,Bj) & \cdots & K_{S2}(B1,Bn) \\ \vdots & \vdots & & \vdots & & \vdots \\ K_{Si}(B1,B1) & K_{Si}(B1,B2) & \cdots & K_{Si}(B1,Bj) & \cdots & K_{Si}(B1,Bn) \\ \vdots & \vdots & & \vdots & & \vdots \\ K_{Sn}(B1,B1) & K_{Sn}(B1,B2) & \cdots & K_{Sn}(B1,Bj) & \cdots & K_{Sn}(B1,Bn) \end{pmatrix} \quad (1)$$

In the matrix A,
Bj represents a j-th (j is an integer of 1 to n) ion among the n kinds of ions.

Si is a symbol representing an i-th (i is an integer of 1 to n) ion-selective electrode constituting the n kinds of ion-selective electrodes.

$K_{Si}(B1, Bj)$ is a selectivity coefficient for the ion Bj when the ion B1 is used as a reference in the ion-selective electrode Si. For example, $K_{S1}(B1, B1)$ represents a selectivity coefficient for the ion B1 when the ion B1 is used as a reference in an ion-selective electrode S1, and $K_{S2}(B1, B3)$ represents a selectivity coefficient for an ion B3 when the ion B1 is used as a reference in an ion-selective electrode S2.

Description of formula (2)

In the present application, a determinant of the matrix A is represented by $|A|$, and an absolute value of the determinant is represented by $\|A\|$. In general, the determinant $|A|$ can be calculated by cofactor expansion, for example.

When three kinds of ions (ions: B1, B2, and B3, ion concentrations: C(B1), C(B2), and C(B3)) in a sample solution are measured using three kinds (n = 3) of ion-selective electrodes S1, S2, and S3, the matrix A represented by the above formula (1) can be represented as a $3 \times 3$ square matrix described below. In this case, a Sallas formula in the matrix can be used, and the determinant $|A|$ corresponding to the matrix A can be determined by formula (12).

[Mathematical formula 13]

$$\text{Matrix A } (3 \times 3) = \begin{pmatrix} K_{S1}(B1,B1) & K_{S1}(B1,B2) & K_{S1}(B1,B3) \\ K_{S2}(B1,B1) & K_{S2}(B1,B2) & K_{S2}(B1,B3) \\ K_{S3}(B1,B1) & K_{S3}(B1,B2) & K_{S3}(B1,B3) \end{pmatrix}$$

$$\begin{aligned}
\text{Determinant } |A| = \ & K_{S1}(B1,B1)K_{S2}(B1,B2)K_{S3}(B1,B3) + K_{S1}(B1,B2)K_{S2}(B1,B3)K_{S3}(B1,B1) \\
& + K_{S1}(B1,B3)K_{S2}(B1,B1)K_{S3}(B1,B2) - K_{S1}(B1,B3)K_{S2}(B1,B2)K_{S3}(B1,B1) \\
& - K_{S1}(B1,B2)K_{S2}(B1,B1)K_{S3}(B1,B3) - K_{S1}(B1,B1)K_{S2}(B1,B3)K_{S3}(B1,B2)
\end{aligned} \tag{12}$$

When the matrix A is an $n \times n$ matrix, the determinant $|A|$ can be easily determined by using spreadsheet software (for example, Microsoft Excel) or numerical calculation software. When spreadsheet software Excel is used, calculation can be performed using a function such as "= MDETERM". If the determinant $|A|$ can be determined, an absolute value $\|A\|$ of the determinant can be calculated.

The most important point in the present invention is that the absolute value $\|A\|$ of the determinant satisfies formula (2).

[Mathematical formula 14]

$$\|A\| > 0.1 \tag{2}$$

<Significance of formula (2)>

From formulas (10) and (11), the following formula (13) can be expressed.

[Mathematical formula 15]

$$A \cdot \begin{pmatrix} C(B1) \\ C(B2) \\ \vdots \\ C(Bn) \end{pmatrix} = \begin{pmatrix} \text{Calculated value obtained with electrode S1} \\ \text{Calculated value obtained with electrode S2} \\ \vdots \\ \text{Calculated value obtained with electrode Sn} \end{pmatrix} \tag{13}$$

When the determinant $|A|$ of the matrix A is not 0, an inverse matrix $A^{-1}$ exists which corresponds to the matrix A. When the inverse matrix $A^{-1}$ is multiplied by each side of formula (13) from the left, formula (13) can be transformed into formula (14). Since $A^{-1} \cdot A$ is an identity matrix, formula (14) can be transformed into formula (15) using a cofactor matrix of the matrix A expressed by formula (16).

[Mathematical formula 16]

$$A^{-1} \cdot A \cdot \begin{bmatrix} C(B1) \\ C(B2) \\ \vdots \\ C(Bn) \end{bmatrix} = A^{-1} \cdot \begin{bmatrix} \text{Calculated value obtained with electrode S1} \\ \text{Calculated value obtained with electrode S2} \\ \vdots \\ \text{Calculated value obtained with electrode Sn} \end{bmatrix} \quad (14)$$

$$\begin{bmatrix} C(B1) \\ C(B2) \\ \vdots \\ C(Bn) \end{bmatrix} = A^{-1} \cdot \begin{bmatrix} \text{Calculated value obtained with electrode S1} \\ \text{Calculated value obtained with electrode S2} \\ \vdots \\ \text{Calculated value obtained with electrode Sn} \end{bmatrix} \quad (3)$$

$$= \frac{\tilde{A}}{|A|} \cdot \begin{bmatrix} \text{Calculated value obtained with electrode S1} \\ \text{Calculated value obtained with electrode S2} \\ \vdots \\ \text{Calculated value obtained with electrode Sn} \end{bmatrix} \quad (15)$$

$$A^{-1} = \frac{\tilde{A}}{|A|} \quad (16)$$

$\overline{A}$ : Cofactor matrix of A

Usually, measurement using a single ion-selective electrode S is affected by a measurement error and an interfering ion. When a calculated value obtained with the ion-selective electrode S includes an error, the error is referred to as $\Delta s$. In the present invention, n kinds of ion-selective electrodes (ion-selective electrodes S1, S2, $\cdots$, and Sn) are used as the ion-selective electrode S, and errors $\Delta s$ for these ion-selective electrodes are referred to as $\Delta_{S1}$, $\Delta_{S2}$, $\cdots$, and $\Delta_{Sn}$, respectively. Formula (15) can be expressed by formula (17). By separating a term indicating an error using a property of a sum of matrices, formula (17) can be expressed by formula (17').
[Mathematical formula 17]

$$\begin{bmatrix} C(B1) \\ C(B2) \\ \vdots \\ C(Bn) \end{bmatrix} = \frac{\tilde{A}}{|A|} \cdot \begin{bmatrix} \text{Calculated value obtained with electrode S1} + \Delta_{S1} \\ \text{Calculated value obtained with electrode S2} + \Delta_{S2} \\ \vdots \\ \text{Calculated value obtained with electrode Sn} + \Delta_{Sn} \end{bmatrix} \quad (17)$$

$$\begin{pmatrix} C(B1) \\ C(B2) \\ \vdots \\ C(Bn) \end{pmatrix} = \frac{\tilde{A}}{|A|} \cdot \begin{pmatrix} \text{Calculated value obtained with electrode S1} \\ \text{Calculated value obtained with electrode S2} \\ \vdots \\ \text{Calculated value obtained with electrode Sn} \end{pmatrix} + \frac{\tilde{A}}{|A|} \cdot \begin{pmatrix} \Delta_{S1} \\ \Delta_{S2} \\ \vdots \\ \Delta_{Sn} \end{pmatrix} \quad (17')$$

$$\underbrace{\hspace{4cm}}_{\text{Term indicating accurate concentration}} \qquad \underbrace{\hspace{3cm}}_{\substack{\text{Term indicating error}}}$$

Since the determinant $|A|$ exists in a denominator of the term indicating an error, a value of the term indicating an error decreases as the absolute value $\|A\|$ of the determinant $|A|$ increases. Specifically, when the absolute value $\|A\|$ of the determinant is 0.1 or more, the value of the term indicating an error is small, and an electrode can be selected with which measurement that can withstand practical use is possible. Therefore, in the present invention, the absolute value $\|A\|$ satisfies a relationship represented by the following formula (2).
[Mathematical formula 18]

$$\|A\| > 0.1 \qquad\qquad (2)$$

The absolute value $\|A\|$ of the determinant is more preferably larger than 0.2, and still more preferably larger than 0.4. The larger the absolute value $\|A\|$ of the determinant is, the better it is. Although there is no upper limit for the absolute value $\|A\|$ of the determinant, it is usually a value of 100 or less.
This formula (2) represents a combination of selectivity coefficients $K_S(B1, Bj)$ (that is, $K_{S1}(Bl, Bj)$, $K_{S2}(B1, Bj)$, $\cdots$, and $K_{Sn}(B1, Bj)$) with which an influence of an error is reduced in relation to n kinds of ion-selective electrodes (ion-selective electrodes S1, S2, $\cdots$, and Sn) used as the ion-selective electrode S and n kinds of ions (ions: B1, B2, $\cdots$, and Bn) in a sample solution. In the present invention, two or more kinds of ion-selective electrodes having mutually different selectivity coefficient ratios for a plurality of ions are used as the ion-selective electrode S, and by selecting n kinds of ion-selective electrodes satisfying formula (2) as the two or more kinds of ion-selective electrodes, it is possible to provide an ion concentration measurement device and a method having a small influence of an error.

Description of formula (3)

When n kinds of ions in a sample solution are measured using the n kinds of ion-selective electrodes (ion-selective electrodes S1, S2, $\cdots$, and Sn) as the ion-selective electrode S, n kinds of ion concentrations can be represented by formula (3).
[Mathematical formula 19]

$$\begin{pmatrix} C(B1) \\ C(B2) \\ \vdots \\ C(Bn) \end{pmatrix} = A^{-1} \cdot \begin{pmatrix} \text{Calculated value obtained with electrode S1} \\ \text{Calculated value obtained with electrode S2} \\ \vdots \\ \text{Calculated value obtained with electrode Sn} \end{pmatrix} \quad (3)$$

Here, $A^{-1}$ represents an inverse matrix of the matrix A, and C(Bj) represents a concentration of the ion Bj (j is an integer of 1 to n). A calculated value obtained with an electrode Si (i is an integer of 1 to n) indicates a value obtained by conversion of a potential difference measured with the ion-selective electrode Si according to the following formula.
[Mathematical formula 20]

$$\text{Calculated value obtained with electrode Si} \quad = \quad 10^{\left(\frac{E_{Si} - E_{0,Si}}{Slope}\right)}$$

In the above formula,

Esi is a potential difference generated between the ion-selective electrode Si and a reference electrode,
$E_{0,Si}$ is a standard potential difference generated between the ion-selective electrode Si and the reference electrode,
Slope is 2.303RT/aF, and
R, T, a, and F are the same as R, T, a, and F defined by the above formula (5), respectively.

In the present invention, the ion-selective electrode S is also referred to as an electrode S, and an i-th (i is an integer of 1 to n) ion-selective electrode Si among n kinds of ion-selective electrodes (ion-selective electrodes S1, S2, ⋯, and Sn) is also referred to as an electrode Si. By using formula (3), an ion concentration of a sample solution can be determined from a calculated value obtained with each of the n kinds of ion-selective electrodes used as the electrode S.

Case of using standard solution

When measurement is performed for a long time, the standard potential difference $E_{0,S}$ may fluctuate depending on a change in measurement conditions such as temperature, and therefore it is preferable to correct the standard potential difference $E_{0,S}$ using a standard solution.

A method for correcting the standard potential difference $E_{0,S}$ using a standard solution will be described. A potential difference obtained when the ion-selective electrode S and the reference electrode are immersed in a standard solution having a known ion concentration ($C_{std}$) is referred to as $E_{std}$ (formula (18)). At this time, the standard potential difference $E_{0,S}$ can be expressed by formula (19).

[Mathematical formula 21]

$$E_{Std} = E_{0,S} + Slope \times \log_{10} C_{std} \qquad (18)$$

$$E_{0,S} = E_{Std} - Slope \times \log_{10} C_{std} \qquad (19)$$

In the above formulas (18) and (19),

Slope is 2.303RT/aF, and
R, T, a, and F are the same as R, T, a, and F defined by the above formula (5), respectively.

When the standard solution is measured and formula (19) is substituted for the standard potential difference $E_{0,S}$, a calculated value obtained with the electrode S can be expressed by formula (20).

[Mathematical formula 22]

$$\text{Calculated value obtained with electrode S} \quad = \quad 10^{\left(\frac{E_S - E_{0,S}}{Slope}\right)}$$

$$= \quad 10^{\left(\frac{E_S - E_{Std}}{Slope} + \log_{10} C_{Std}\right)} \qquad (20)$$

It is preferable to use formula (20) for the calculated value obtained from the electrode S because measurement can be performed while fluctuation of the standard potential difference $E_{0,S}$ is corrected when measurement conditions such as temperature change or when measurement is performed for a long time.

In the present invention, n kinds of ion-selective electrodes (ion-selective electrodes S1, S2, ⋯, and Sn) are used as the electrode S. Therefore, by applying the above formula (20) to each of calculated values obtained from these ion-selective electrodes, it is possible to perform measurement similarly while fluctuation of the standard potential difference

$E_{0,S}$ (that is, $E_{0,S1}$, $E_{0,S2}$, $\cdots$, and $E_{0,Sn}$) for these ion-selective electrodes is corrected. For example, when the above formula (20) is applied to an i-th (i is an integer of 1 to n) ion-selective electrode Si among n kinds of ion-selective electrodes (ion-selective electrodes S1, S2, $\cdots$, and Sn), the above formula (20) can be expressed as the following formula (20'). [Mathematical formula 23]

$$\text{Calculated value obtained with electrode Si} = 10^{\left( \dfrac{E_{Si} - E_{0,Si}}{\text{Slope}} \right)}$$

$$= 10^{\left( \dfrac{E_{Si} - E_{Stdi}}{\text{Slope}} + \log_{10} C_{Std} \right)} \quad (20')$$

In the above formula (20'),

$E_{Si}$ is a potential difference generated between the ion-selective electrode Si and a reference electrode,

$E_{0,Si}$ is a standard potential difference generated between the ion-selective electrode Si and the reference electrode,

$E_{Stdi}$ is a potential difference generated between the ion-selective electrode Si and the reference electrode when the ion-selective electrode Si and the reference electrode are immersed in a standard solution having a known ion concentration ($C_{std}$),

Slope is 2.303RT/aF, and

R, T, a, and F are the same as R, T, a, and F defined by the above formula (5), respectively.

[Ion concentration measurement device]

The ion concentration measurement device of the present invention includes two or more kinds of ion-selective electrodes that have mutually different selectivity coefficient ratios for a plurality of ions and satisfy the above formulas (1) and (2). Here, when the number of kinds of the plurality of ions is referred to as n, the two or more kinds of ion-selective electrodes are constituted by n kinds of ion-selective electrodes, and n is an integer of 2 or more.

In addition, in the above formula (1),

Bj represents a j-th ion among n kinds of ions constituting the plurality of ions,

Si is a symbol representing an i-th ion-selective electrode constituting the n kinds of ion-selective electrodes,

i and j are each independently an integer of 1 to n, and

$K_{Si}(B1, Bj)$ represents an ion selectivity coefficient for the ion Bj when an ion B1 is used as a reference in the ion-selective electrode Si.

In the present invention, it is preferable to calculate an ion concentration by the above formula (3).

Here, in the present invention, an ion B1 (that is, an ion satisfying j = 1 among ions Bj) is one ion appropriately selected from n kinds of ions constituting the plurality of ions. This ion B1 is usually an ion that is often selected as an ion to be measured in concentration measurement by a single ion-selective electrode.

On the other hand, ions other than the ion B1 among the n kinds of ions constituting the plurality of ions are referred to as ions B2, B3, $\cdots$, and Bn (that is, ions satisfying j $\geq$ 2). Here, the ions B2, B3, $\cdots$, and Bn may include an ion whose concentration is difficult to directly and selectively measure by a single ion-selective electrode.

With respect to the two or more kinds of ion-selective electrodes, in the present invention, as long as a combination of ion-selective electrodes selected as n kinds of ion-selective electrodes (ion-selective electrodes S1, S2, $\cdots$, and Sn) satisfies the above formula (2), assignment of the symbols S1, S2, $\cdots$, and Sn is not particularly limited, and these symbols can be appropriately assigned to these ion-selective electrodes. Here, the ion concentration measurement device of the present invention may include more than n kinds of ion-selective electrodes, and n kinds of ion-selective electrodes may be selected from these ion-selective electrodes so as to satisfy formula (2) and used for ion concentration measurement. To each of the ion-selective electrodes constituting the ion concentration measurement device of the present invention, any determined unique identifier may be attached separately from the symbol Si. In this case, a correspondence table between these identifiers and the symbols S1, S2, $\cdots$, and Sn is prepared according to components that can be contained in a sample solution and the kinds of ion-selective electrodes, and on the basis of this correspondence table, ion-selective electrodes to be used as n kinds of ion-selective electrodes (ion-selective electrodes S1, S2, $\cdots$, and Sn) may be selected from the ion-selective electrodes constituting the ion concentration measurement device.

The ion concentration measurement device in the present invention is not particularly limited as long as it is a device including two or more kinds of ion-selective electrodes that have mutually different selectivity coefficient ratios for a plurality of ions and satisfy formulas (1) and (2). Specific examples thereof include a device as illustrated in Fig. 1. Usually, the ion concentration measurement device includes an ion-selective electrode 1, a reference electrode 2, and a potentiometer 3. Here, each of ion-selective electrodes 1 functions as the ion-selective electrode S. The ion-selective electrodes 1 are connected to potentiometers 3 via an electric wire, respectively, and the potentiometers 3 are connected to a reference electrode 2 via an electric wire. That is, each of the n kinds of ion-selective electrodes (ion-selective electrodes S1, S2, ⋯, and Sn) used as two or more kinds of ion-selective electrodes constitutes the ion-selective electrode 1 in Fig. 1. In addition, it is also possible to combine an arithmetic device capable of calculating the above-described formulas and a tank 4 to contain a solution. Here, the arithmetic device is connected to the potentiometer 3, and performs arithmetic processing such as calculation of an ion concentration on the basis of a potential difference input from the potentiometer 3. The arithmetic processing can include a step of forming the determinant $|A|$ and a step of calculating an absolute value of the determinant $|A|$. The arithmetic device and the potentiometer 3 may be separate devices, or may be integrated in a single device. These are examples, and do not limit the form of the device.

The number of kinds of ion-selective electrodes 1 to be combined may be any number as long as it is two or more. The number of kinds of ion-selective electrodes 1 only needs to be appropriately adjusted according to the number of kinds of ions to be measured, but is preferably 2 or more and 10 or less from a viewpoint of measurement precision. The number of kinds of ion-selective electrodes 1 is most preferably 2 or more and 5 or less from a viewpoint of the size of the ion concentration measurement device. In other words with the n, the n is preferably 2 or more and 10 or less, and most preferably 2 or more and 5 or less. Since it is necessary to use two or more kinds of ion-selective electrodes, the number of ion-selective electrodes 1 needs to be two or more. There is no particular limitation as long as it is two or more. In an exemplary aspect of the present invention, the number of kinds of the ion-selective electrodes 1 is three (that is, n = 3), but is not limited thereto. It is also possible to combine a plurality of ion-selective electrodes of the same type.

The number of the reference electrodes 2 is not particularly limited as long as it is one or more, but is usually smaller than the number of the ion-selective electrodes.

The potentiometer 3 is not limited as long as it can measure a potential difference between the two or more kinds of ion-selective electrodes and the reference electrode. A voltmeter can also be used as long as it can determine a potential difference.

The tank 4 to contain a solution can be used without particular limitation as long as the ion-selective electrode and the reference electrode can be immersed therein. A plurality of tanks 4 may be used according to the number of kinds of ion-selective electrodes, or the plurality of ion-selective electrodes may be immersed in one tank. A tank in which each electrode can be immersed in a batch manner may be used, or a channel may be formed to circulate a sample solution, for example. In addition, a sample solution may also be stirred or circulated in order to homogenize the sample solution.

Ion-selective electrode

The ion-selective electrode 1 functions as the ion-selective electrode S, and any ion-selective electrode that can be generally used, such as an internal liquid type ion-selective electrode or a solid electrode type ion-selective electrode, can be used without particular limitation. In the present invention, n kinds of ion-selective electrodes 1 having mutually different selectivity coefficient ratios for a plurality of ions are combined and used as the ion-selective electrodes S1, S2, ⋯, and Sn.

As illustrated in Fig. 1, the internal liquid type ion-selective electrode includes an internal electrode 5, an internal liquid 6, and an ion-selective membrane 7, and the ion-selective membrane 7 and the sample solution 9 are in contact with each other.

The internal electrode 5 is not particularly limited as long as it is a usually used one. A silver/silver chloride electrode is usually used.

As the internal liquid 6, an aqueous solution of an electrolyte such as KCl or NaCl is usually used.

The ion-selective membrane 7 is not particularly limited, but a substrate of a polymer such as polyvinyl chloride is mixed with a ligand that selects an ion together with a plasticizer, and the resultant mixture is used. The ion-selective membrane 7 may contain a salt, for example. Specifically, when the ion-selective electrode 1 is a cation-selective electrode for, for example, sodium, potassium, calcium, or magnesium (that is, a cation-selective electrode that selectively reacts with a cation such as sodium, potassium, calcium, or magnesium to generate an output corresponding to a concentration (activity) of the cation), a membrane as described in Pure Appl. Chem., 2000, Vol. 72, No. 10, pp. 1851-2082 can be used as the ion-selective membrane 7. When the ion-selective electrode 1 is an anion-selective electrode for, for example, chloride, carbonate, thiocyanate, nitrate, hydroxide, phosphate, sulfate, or iodide (that is, an anion-selective electrode that selectively reacts with an anion such as nitrate, hydroxide, phosphate, sulfate, or iodide to generate an output corresponding to a concentration (activity) of the anion), a membrane as described in Pure Appl. Chem., 2002, Vol. 74, No. 6, pp. 923-994, and a silver halide such as silver chloride or silver bromide or an ion-exchange membrane (JP H10-318973 A, JP H11-132991 A, and JP 2003-207476 A) can be used as the ion-selective membrane 7. A membrane as described above can be prepared by a user and used, or a commercially available membrane can be used. That is, the ion-selective membrane 7 may be prepared by a user or may be a commercially available membrane as long as the ion-selective membrane 7 is a membrane as described above. Examples of the commercially available membrane include Neoceptor (registered trademark) CSE (registered trademark) (manufactured by ASTOM CORPORATION) and Neoceptor ASE (manufactured by ASTOM CORPORATION).

Selection of ion-selective electrode

In the present invention, it is necessary to select two or more kinds of ion-selective electrodes satisfying formulas (1) and (2) as an ion-selective electrode constituting the ion concentration measurement device. Whether formulas (1) and (2) are satisfied is determined by a selectivity coefficient $K_S(B1, Bj)$ of each of the ion-selective electrodes.

The ion selectivity coefficient $K_S(B1, Bj)$ can be generally calculated by a single solution method or a mixed solution method. In the mixed solution method, a potential difference for a mixed solution containing an ion B1 having a known concentration and an ion Bj for which a selectivity coefficient is to be determined is measured, and the ion selectivity coefficient $Ks(B1, Bj)$ is calculated.

An example of such calculation is described with a specific numerical value when a mixed solution containing the ion B1 and a second ion B2 is measured using two kinds of ion-selective electrodes (a first ion-selective electrode S1 and a second ion-selective electrode S2 as the ion-selective electrode S. It is assumed that ion selectivity coefficients $K_S(B1, B2)$ for the ion B2 when the ion B1 is used as a reference in the ion-selective electrodes S1 and S2 are $K_{S1}(B1, B2) = 0.5$ and $K_{S2}(B1, B2) = 0.1$, respectively. Here, the ion selectivity coefficients B1) for the ion B1 itself when the ion B1 is used as a reference for the ion-selective electrodes S1 and S2 can be regarded as $K_{S1}(B1, B1) = 1$ and $K_{S2}(B1, B1) = 1$, respectively. The matrix A and the determinant |A| can be expressed by the following formulas using these selectivity coefficients, respectively. In this case, the determinant |A| = -0.4 can be obtained by calculation.

[Mathematical formula 24]

$$\text{Matrix A} = \begin{pmatrix} K_{S1}(B1,B1) & K_{S1}(B1,B2) \\ K_{S2}(B1,B1) & K_{S2}(B1,B2) \end{pmatrix}$$

$$\begin{aligned} \text{Determinant } |A| &= K_{S1}(B1,B1)K_{S2}(B1,B2) - K_{S1}(B1,B2)K_{S2}(B1,B1) \\ &= K_{S2}(B1,B2) - K_{S1}(B1,B2) \\ &= 0.1 - 0.5 = -0.4 \end{aligned}$$

Reference electrode

The reference electrode 2 is not particularly limited as long as it is a usually used one. Note that, in a typical aspect, as illustrated in Fig. 1, the reference electrode 2 includes the internal electrode 5, the internal liquid 6, and a liquid junction 8. Examples of the reference electrode 2 that can be used in the present invention include a silver-silver chloride reference electrode using a 3 M aqueous potassium chloride solution as the internal liquid 6, using a silver-silver chloride electrode as the internal electrode 5, and having the liquid junction 8 between a sample solution and the internal liquid. Note that the reference electrode 2 may be another reference electrode, for example, a saturated calomel electrode.

Ion

Any ion can be used as long as it can prepare a solution of the ion and can be measured by a device. The ion may be an anion or a cation, and examples of the anion include a chloride ion, a bicarbonate ion, an anion of an organic acid, a nitrate ion, and a sulfate ion, and examples of the cation include a potassium ion, a silver ion, and a copper ion. More preferable examples thereof include a plurality of ions (a chloride ion, a bicarbonate ion, and an anion of an organic acid such as lactic acid, pyruvic acid, propionic acid, fatty acid, or amino acid, for example) contained in blood from a viewpoint that the characteristics of the present device can be most utilized.

Here, the "plurality of ions" used in the present invention may be two or more kinds of anions or two or more kinds of cations. When the "plurality of ions" are anions, the "plurality of ions" may be two or more selected from the anions exemplified above, and for example, may contain a chloride ion and a bicarbonate ion, preferably a chloride ion, a bicarbonate ion, and an anion of an organic acid. In one preferable and exemplary aspect of the present invention, the "plurality of ions" are a chloride ion, a bicarbonate ion, and an acetate ion. Note that the "plurality of ions" used in the present invention are not limited to those exemplified above, and may be appropriately selected according to a measurement target.

Here, in the present invention, when evaluation using the above formulas (1) and (2) is performed, one ion which is the ion B1 (that is, an ion satisfying j = 1 among ions Bj) is selected from n kinds of ions constituting the "plurality of ions", and the other n-1 kinds of ions are defined as ions B2, B3, $\cdots$, and Bn (that is, ions satisfying j $\geq$ 2 among ions Bj).

Among these ions, the ion B1 is usually an ion whose concentration can be directly measured using one type of ion-selective electrode having an extremely small selectivity coefficient for another ion singly. Examples of an ion that can be the ion B1 include a chloride ion and a sodium ion.

On the other hand, the ions other than the ion B1 (that is, ions B2, B3, $\cdots$ and Bn) may be ions whose concentration can be directly measured by one single type of ion-selective electrode, or may be ions whose concentrations are difficult to directly measure by one single type of ion-selective electrode. Examples of an ion that can be an ion other than the ion B1 include a bicarbonate ion and an anion of an organic acid, such as an acetate ion.

According to the present invention, it is possible to measure a concentration of even an ion for which it is difficult to obtain an electrode having a sufficiently small ion selectivity coefficient for another ion, such as a bicarbonate ion. Based on this fact, in one preferable and exemplary aspect of the present invention, the ion B1 is a chloride ion, and ions other than the ion B1 include a bicarbonate ion.

[Ion concentration measurement method]

An ion concentration measurement method of the present invention includes a step of measuring an ion concentration in a sample solution using a plurality of ion-selective electrodes having mutually different selectivity coefficient ratios for a plurality of ions and satisfying the above formulas (1) and (2). Here, when the number of kinds of the plurality of ions is referred to as n, the two or more kinds of ion-selective electrodes are constituted by n kinds of ion-selective electrodes, and n is an integer of 2 or more.

In addition, in the above formula (1),

Bj represents a j-th ion among n kinds of ions constituting the plurality of ions,
Si is a symbol representing an i-th ion-selective electrode constituting the n kinds of ion-selective electrodes,
i and j are each independently an integer of 1 to n, and
$K_{Si}$(B1, Bj) represents an ion selectivity coefficient for the ion Bj when an ion B1 is used as a reference in the ion-selective electrode Si.

In the present invention, it is preferable to calculate an ion concentration by the above formula (3).

The ion concentration measurement method of the present invention is not particularly limited regarding, for example, a tool or a device to be used as long as it is an ion concentration measurement method using a plurality of ion-selective electrodes having mutually different selectivity coefficient ratios for a plurality of ions and satisfying the above formulas (1) and (2), but for example, it is convenient to use an ion concentration measurement device as described above.

A concentration of an ion (the ion B1) to be measured is not particularly limited, but is preferably 0 mM to 1 M. The concentration is more preferably 0 mM to 300 mM.

When measurement is performed for a long time, the standard potential difference $E_{0,S}$ may fluctuate depending on a change in measurement conditions such as temperature, and therefore it is preferable to correct the standard potential difference $E_{0,S}$ using a standard solution.

First, the sample solution and the standard solution will be described.

Sample solution

<Sample>

A sample is not particularly limited, and any sample containing an ion when the sample is made into a solution can be measured. In particular, the effect of the present invention is high in a sample containing a plurality of kinds of ions, such as a biological sample (blood, serum, plasma, for example). A sample may be measured as it is depending on a state or a concentration of the sample and a coexisting substance, but preferably, the sample is diluted with a buffer, water, an organic solvent, for example, in order to suppress fluctuation of pH and to eliminate a liquid junction potential, and measured as a sample solution. A sample can be used after being pretreated with, for example, a column or a filter depending on a coexisting substance.

<Buffer>

It is not necessary to use a buffer as long as an ion concentration can be stably measured, but it is preferable to use the buffer because fluctuation of pH is suppressed and a liquid junction potential is eliminated.

As the buffer, a generally known buffer can be used without limitation as long as the above object can be achieved. Specific examples thereof include a type of buffer called Good's Buffer (a HEPES buffer, a MES buffer, and an ADA buffer) and a Tris buffer. For example, in a case of measurement of a bicarbonate ion, a HEPES buffer is preferable from a viewpoint that a buffer that makes a response inhibition of an ion-selective electrode to an ion small is preferable. The buffer has pH of about 6.0 to 8.0 in a case of Good's Buffer. A concentration of the buffer is not particularly limited, but is preferably 1 mM to 1 M. A buffer having a concentration of 1 mM to 500 mM is more preferable. A buffer having a concentration of 5 mM to 300 mM is still more preferable.

Standard solution

The standard solution is an ion solution having a known concentration, used to correct the standard potential difference $E_{0,S}$. An ion used for the standard solution is not particularly limited as long as a potential ($E_{std}$) can be measured. Two or more kinds of ions can be mixed and used for the standard solution. Nevertheless, it is usually convenient to select one kind from measurement ions and use it for the standard solution, which is preferable.

A solvent used for the standard solution is not limited as long as a potential can be measured, and examples thereof include water and an organic solvent. Two or more kinds of solvents may be mixed and used. Water is preferably used as the solvent from a viewpoint of stability of pH. A buffer is more preferably used in order to suppress fluctuation of pH and to eliminate a liquid junction potential. As the buffer, those that can be used for the sample solution as described above can be used.

An ion concentration ($C_{std}$) of the standard solution is not particularly limited, and only needs to be appropriately determined according to a measurement purpose. Usually, the ion concentration ($C_{std}$) is 0.1 to 10 mM. More preferably, the ion concentration is 0.5 to 3 mM.

Measurement of potential difference and calculation of ion concentration

An ion concentration measurement method will be described, but it is an example and is not particularly limited.

In general, a potential difference is measured, values of $E_{0,S}$ and Slope are determined, and a calculated value obtained with the electrode S is determined from these values. Then, an ion concentration is calculated from the calculated value obtained with the electrode S.

Calculation of an ion concentration in a case of using the standard solution will also be described, but it is one of means for performing measurement with high precision, and does not limit the present invention.

<Measurement of potential difference $E_S$ of sample solution>

The potential difference Es is measured by immersing an ion-selective electrode and a reference electrode in a sample solution and measuring a potential difference with a potentiometer. Measurement conditions such as a measurement temperature and a measurement time are not particularly limited as long as the potential difference Es can be measured. The measurement temperature is preferably 20°C to 45°C, and the measurement time is preferably 2 seconds to 15 minutes from a viewpoint that a stable potential difference can be measured.

<Creation of calibration curve (calculation of values of $E_{0,S}$ and Slope) >

By measuring a potential difference Es of a known solution containing a certain ion B1 at an ion concentration C(B1), a calibration curve is created from a Nernst equation, and values of $E_{0,S}$, and Slope can be determined.

[Mathematical formula 25] $E_S = E_{0,S} + \text{Slope} \times \log_{10} C(B1)$

In the above formula,

Slope is 2.303RT/aF, and
R, T, a, and F are the same as R, T, a, and F defined by the above formula (5), respectively.

As an ion used for the calibration curve, any one type of ion among ions to be measured is usually used. Here, the ion used for the calibration curve is described as B1.

A solution having a known concentration for creating the calibration curve preferably has a composition close to that of a sample solution to be measured from a viewpoint of increasing precision of the calibration curve. Therefore, a solvent of the sample solution and a solvent of the solution used for the calibration curve are preferably the same. An ion concentration $C(B1)$ only needs to be appropriately determined according to the sample solution to be measured. The calibration curve can be created by measuring ion concentrations at two different points, but the calibration curve is created by measuring the ion concentration $C(B1)$ at three or more points from a viewpoint of increasing the precision.

<Calculation of calculated value obtained with electrode S and ion concentration>

Using a potential difference $E_S$ between an ion-selective electrode and a reference electrode, obtained by immersing them in a sample solution, and the calibration curve as described above, a calculated value obtained with the electrode S can be calculated by the following formula using the determined values of $E_{0,S}$ and Slope.

[Mathematical formula 26]

$$\text{Calculated value obtained with electrode S} = 10^{\left(\dfrac{E_S - E_{0,S}}{Slope}\right)}$$

For n kinds of ion-selective electrodes S1 to Sn used as the electrode S, the "calculated values obtained with the electrode S" are determined and defined as "a calculated value obtained with the electrode S1", "a calculated value obtained with the electrode S2", ···, and "a calculated value obtained with the electrode Sn", respectively. By using these calculated values and an inverse matrix $A^{-1}$ of a matrix A constituted by selectivity coefficients of the ion-selective electrodes used, ion concentrations ($C(B1)$, $C(B2)$, ···, and $C(Bn)$) of n kinds of ions (ions: B1, B2, ···, and Bn) can be determined.

[Mathematical formula 27]

$$
\begin{pmatrix} C(B1) \\ C(B2) \\ \vdots \\ C(Bn) \end{pmatrix} = A^{-1} \cdot \begin{pmatrix} \text{Calculated value obtained with electrode S1} \\ \text{Calculated value obtained with electrode S2} \\ \vdots \\ \text{Calculated value obtained with electrode Sn} \end{pmatrix} \quad (3)
$$

<Calculation of ion concentration in case of using standard solution>

When the standard solution is used, the ion-selective electrode and the reference electrode are immersed in the standard solution, the potential difference $E_{std}$ is determined, and the standard potential difference $E_{0,S}$ is corrected. A tool or a device, for example, to be used is not particularly limited as long as the potential difference $E_{std}$ can be measured, but it is convenient to use a similar device to that for measuring the sample solution.

In measurement of the standard solution, a measurement temperature is preferably 20°C to 45°C, and a measurement time is preferably 2 seconds to 15 minutes from a viewpoint that a stable potential difference can be measured.

By once measuring the potential difference $E_{std}$ of the standard solution, correction can be performed. However, in order to determine the calculated value obtained with the electrode S with higher precision, it is preferable to repeatedly measure the potential difference $E_{std}$ between measurements of the sample solution. An interval at which the potential difference $E_{std}$ of the standard solution is repeatedly measured only needs to be appropriately determined. When the measurement is repeatedly performed, a potential difference $E_{std}$ at any time point may be adopted, and correction may be performed. Potential differences $E_{std}$ at several points may be averaged and used for correction. It is preferable to adopt a potential difference $E_{std}$ measured 2 seconds to 1 hour before measuring the potential difference $E_S$ of the target sample solution from a viewpoint of precision.

Using the potential difference $E_{std}$ obtained when the electrode S is immersed in the standard solution and the ion concentration ($C_{std}$) of the standard solution, a calculated value obtained with the electrode S can be calculated by formula (20).

[Mathematical formula 28]

$$\text{Calculated value obtained with electrode S} = 10^{\left(\dfrac{E_S - E_{0,S}}{\text{Slope}}\right)}$$

$$= 10^{\left(\dfrac{E_S - E_{Std}}{\text{Slope}} + \log_{10} C_{Std}\right)} \quad (20)$$

For n kinds of ion-selective electrodes S1 to Sn used as the electrode S, the "calculated values obtained with the electrode S" are determined and defined as "a calculated value obtained with the electrode S1", "a calculated value obtained with the electrode S2", $\cdots$, and "a calculated value obtained with the electrode Sn", respectively. By using these calculated values and an inverse matrix $A^{-1}$ of a matrix A constituted by selectivity coefficients of the ion-selective electrodes used, ion concentrations (C(B1), C(B2), $\cdots$, and C(Bn)) of n kinds of ions (ions: B1, B2, $\cdots$, and Bn) can be determined.

EXAMPLES

Hereinafter, the present invention will be described in more detail with reference to Examples, but the present invention is not limited in any way by these Examples.

[Configuration of ion concentration measurement device]

An ion concentration measurement device used in the following Examples and Comparative Example includes an ion-selective electrode, a reference electrode, a potentiometer, a tank to contain a solution, and an arithmetic device.
The ion-selective electrode is connected to the potentiometer via an electric wire, and the potentiometer is connected to the reference electrode via an electric wire. In addition, the potentiometer is connected to the arithmetic device. In the ion concentration measurement, a liquid to be measured (for example, a sample solution, a standard solution, or a calibration curve solution described later) is introduced into the "tank to contain a solution", and the ion concentration measurement is performed in a state where a portion having the ion-selective electrode in the ion-selective electrode and a portion having a liquid junction in the reference electrode are immersed in the "liquid to be measured".
In the following Examples and Comparative Example, as the reference electrode, the potentiometer, the tank to contain a solution, and the arithmetic device, those described below were used unless otherwise specified. Note that the ion-selective electrodes used in the following Examples and Comparative Example will be described later in the following Examples and Comparative Example.

(Reference electrode)

As the reference electrode, a commercially available silver-silver chloride reference electrode (218087) manufactured by A & T Co., Ltd. was used.

(Potentiometer, arithmetic device, and tank to contain a solution)

As the potentiometer, the arithmetic device, and the tank to contain a solution, those attached to an electrolyte analyzer ELA08 of A & T Co., Ltd. were used.

[Preparation of ion-selective electrode]

As the ion-selective electrode, an electrode obtained by modifying a commercially available chloride ion electrode (manufactured by A & T Co., Ltd.) such that an ion-selective membrane was replaceable was used.

The ion-selective membrane was prepared by changing a blending ratio of an amine and a halide and a reaction time as presented in Table 1 on the basis of the method described in Example 1 of JP 2018-004633 A. At this time, trimethylamine was used as a tertiary amine. The reaction time was appropriately adjusted between 12 hours and 24 hours, to give seven kinds of ion-selective membranes having mutually different ion selectivities.

Ion-selective electrodes obtained using these seven kinds of ion-selective membranes (represented by electrode symbols EL1 to EL7, respectively) were used for ion concentration measurement in the following Examples and Comparative Example. Here, in the following description, the ion-selective electrodes of the electrode symbols EL1 to EL7 may be referred to as electrodes EL1 to EL7, respectively. Note that the following electrode symbols EL1 to EL7 correspond to unique identifiers separately determined from the symbols S1 to Sn of the ion-selective electrode for the matrix A.

[Table 1]

| Electrode symbol | Ratio of amine:halide | Reaction time (hour) |
|---|---|---|
| EL1 | 1:9 | 12 |
| EL2 | 1:1 | 24 |
| EL3 | 2:1 | 15 |
| EL4 | 1:2 | 12 |
| EL5 | 1:1 | 20 |
| EL6 | 2:1 | 24 |
| EL7 | 1:2 | 24 |

[Example 1]

1-1 Configuration of ion concentration measurement device

In the present Example, as an ion concentration measurement device, a device having the configuration described in the above "Configuration of ion concentration measurement device" was used.

(Ion-selective electrode)

As ion-selective electrodes, the ion-selective electrode of the electrode symbol EL1 ("electrode EL1"), the ion-selective electrode of the electrode symbol EL2 ("electrode EL2"), and the ion-selective electrode of the electrode symbol EL3 ("electrode EL3") obtained in the above "Preparation of ion-selective electrode" were used.

(Reference electrode, potentiometer, arithmetic device, and tank to contain a solution)

As a reference electrode, a potentiometer, an arithmetic device, and a tank to contain a solution, those described in the above "Configuration of ion concentration measurement device" were used.

In the device used in Examples, potential differences $E_S$ of the plurality of ion-selective electrodes with respect to a solution introduced into the tank to contain a solution can be simultaneously measured in parallel. Therefore, in Examples, the potential differences of the plurality of ion-selective electrodes with respect to a solution were simultaneously measured, but this does not limit the present invention. Any method capable of obtaining a potential difference between a plurality of ion-selective electrodes with respect to the same solution can be used without particular limitation, and the potential differences obtained for the ion-selective electrodes may be measured simultaneously or in any order.

1-2 Sample solution

Since anions in blood are $Cl^-$ > $HCO_3^-$ > an ion of an organic acid (lactic acid, pyruvic acid, propionic acid, fatty acid, amino acid, for example) (here, an acetate ion ($AcO^-$) is used instead) in descending order, these three anions were used as ions to be measured. Since blood contains a contaminant ion in addition to these anions, a solution obtained by adding a phosphate ion, a bromine ion, and a thiocyanate ion was used as a sample solution. A salt containing an ion to be measured and a contaminant substance ion presented in Table 2 was dissolved in a HEPES buffer (100 mM, pH 8.0), to give a sample solution X.

[Table 2]

| | | Sample solution/mM |
|---|---|---|
| Measurement target | NaHCO$_3$ | 24 |
| | NaCl | 105 |
| | NaOAc | 6 |
| Contaminant substance | NaH$_2$PO$_4$ | 1 |
| | NaBr | 1 |
| | NaSCN | 2 |

A solvent is a HEPES buffer (100 mM, pH 8.0)

1-3 Standard solution

In 1 L of HEPES buffer (100 mM, pH 8.0), 0.58 g of sodium chloride (NaCl) was dissolved, to give a 1 mM (= $C_{Std}$) sodium chloride (NaCl)/HEPES buffer.

1-4 Calibration curve solution

Sodium chloride (NaCl) was dissolved in a HEPES buffer (100 mM, pH 8.0) to give 80, 100, and 120 mM sodium chloride (NaCl)/HEPES buffers, and these buffers were used as calibration curve solutions.

1-5 Creation of calibration curve (calculation of values of $E_{std}$ and Slope)

A calibration curve was created as follows.
In a vial, 2 ml of a calibration curve solution was put, the vial was set in an electrolyte analyzer ELA08 manufactured by A & T Co., Ltd., and measurement was performed at room temperature. The measurement was performed by first measuring a potential difference $E_{Std}$ of the standard solution prepared in the above section 1-3 for one calibration curve solution using the ion-selective electrode (the electrode EL1, the electrode EL2, or the electrode EL3) and the reference electrode connected to each other via the potentiometer, and then measuring a potential difference $E_S$ of the calibration curve solution. Such a series of operations was performed for each of the three kinds of calibration curve solutions. Here, when the standard solution is set in a predetermined place, the standard solution is automatically circulated, and the potential $E_{std}$ of the standard solution is measured as needed.
Table 3 presents a potential difference Es when each of the calibration curve solutions of 80, 100, and 120 mM (= C(Cl$^-$)) prepared in the above section 1-4 was measured with each of the electrodes EL1, EL2, and EL3, and a potential difference $E_{Std}$ of the standard solution.
A value of Slope was determined as follows.
From formulas (18') and (19), the following formula (X1) is obtained. The above values were substituted into this formula (X1) to calculate a potential difference ($E_s$ - $E_{Std}$) and ($\log_{10}$C (Cl$^-$) - $\log_{10}C_{std}$), and a calibration curve was prepared to determine Slope. Table 3 presents Slope of each of the electrodes.
[Mathematical formula 29]

$$E_S = E_{0,S} + Slope \times \log_{10} C(Cl^-) \qquad (18')$$

$$E_{0,S} = E_{Std} - Slope \times \log_{10} C_{std} \qquad (19)$$

$$E_S - E_{Std} = Slope \times \left[ \log_{10} C(Cl^-) - \log_{10} C_{std} \right] \qquad (X1)$$

[Table 3]

| Table 3 Potential and Slope value obtained by measuring calibration curve solution using electrode EL1, EL2, or EL3 as electrode S | | | | | | |
|---|---|---|---|---|---|---|
| Electrode symbol | | EL1 | | EL2 | | EL3 | |
| Calibration curve solution | | Es/mV | Estd/mV | Es/mV | Estd/mV | Es/mV | Estd/mV |
| Cl⁻                          80 mM | | 52.31 | 146.27 | 51.85 | 144.55 | 50.37 | 144.33 |
| Cl⁻                         100 mM | | 46.77 | 145.51 | 46.42 | 143.84 | 44.83 | 143.57 |
| Cl⁻                         120 mM | | 42.73 | 145.38 | 42.52 | 143.80 | 40.81 | 143.46 |
| Slope/(mV/dec) | | -49.37 | | -48.71 | | -49.37 | |

1-6 Calculation of selectivity coefficient and creation of matrix A for ion-selective electrode

(Calculation of selectivity coefficient)

For each of the electrodes (electrodes EL1 to EL3) of the electrode symbols EL1 to EL3, a selectivity coefficient for an ion to be measured was determined. Here, an example in which a chloride ion ($Cl^-$) is adopted as the ion to be measured is described.
The selectivity coefficient was calculated as follows.

(1) Calculation of bicarbonate ion ($HCO_3^-$) selectivity coefficient

First, a bicarbonate ion ($HCO_3^-$) selectivity coefficient calculating solution containing an ion (ion to be measured) (chloride ion ($Cl^-$)) as a reference for creating a calibration curve and an ion (bicarbonate ion ($HCO_3^-$)) whose selectivity coefficient was to be calculated was prepared.
Sodium bicarbonate ($NaHCO_3$) and sodium chloride ($NaCl$) were dissolved in a HEPES buffer (100 mM, pH 8.0), to give a 10 mM sodium bicarbonate ($NaHCO_3$) and 100 mM sodium chloride ($NaCl$)/HEPES buffer, and the 10 mM sodium bicarbonate ($NaHCO_3$) and 100 mM sodium chloride ($NaCl$)/HEPES buffer was used as the bicarbonate ion ($HCO_3^-$) selectivity coefficient calculating solution.
In a vial, 2 ml of the bicarbonate ion ($HCO_3^-$) selectivity coefficient calculating solution was put, the vial was set in an electrolyte analyzer ELA08 manufactured by A & T Co., Ltd., and measurement was performed at room temperature. The measurement was performed by first measuring a potential difference $E_{Std}$ of the standard solution for one selectivity coefficient calculating solution using the ion-selective electrode (the electrode EL1, the electrode EL2, or the electrode EL3) and the reference electrode connected to each other via the potentiometer, and then measuring a potential difference Es of the selectivity coefficient calculating solution.
Here, into the following formula (19),
[Mathematical formula 30]

$$E_{0,S} = E_{Std} - Slope \times \log_{10} C_{std} \qquad (19)$$

a potential difference $E_{Std}$ at the time of measuring the standard solution, a value of Slope described in the above Table 3, obtained in the above "1-5 Creation of calibration curve", and an ion concentration $C_{Std}$ of the standard solution are substituted to determine a standard potential difference $E_{0,S}$. Furthermore, into the following formula (8B),
[Mathematical formula 31]

$$K_S(B1,B2) = \frac{10^{\left(\frac{E_S - E_{0,S}}{Slope}\right)} - C(B1)}{C(B2)} \qquad (8B)$$

$$= \frac{10^{\left[\dfrac{E_S - E_{Std}}{Slope} + \log_{10} C_{Std}\right]} - C(B1)}{C(B2)} \quad (8\,B')$$

the standard potential difference $E_{0,S}$, the potential difference $E_S$, the value of Slope, and an ion concentration (A concentration of a chloride ion (Cl$^-$) is represented as C(B1), and a concentration of a bicarbonate ion (HCO$_3^-$) is represented as C(B2).) in the bicarbonate ion (HCO$_3^-$) selectivity coefficient calculating solution are substituted, whereby a selectivity coefficient (Ks(Cl$^-$, HCO$_3^-$)) for the bicarbonate ion (HCO$_3^-$) when the chloride ion (Cl$^-$) is used as an ion to be measured is obtained.

Table 4 presents the potential difference $E_S$, the potential difference $E_{Std}$ of the standard solution, and the selectivity coefficient (Ks(Cl$^-$, HCO$_3^-$)) when the bicarbonate ion (HCO$_3^-$) selectivity coefficient calculating solution is measured with each of the electrodes EL1, EL2, and EL3.

[Table 4]

| Table 4 Measured values of potential difference Es and potential difference $E_{Std}$ of standard solution, and bicarbonate ion (HCO$_3^-$) selectivity coefficient when electrode EL1, EL2, or EL3 is used as electrode S | | | | | | |
|---|---|---|---|---|---|---|
| Electrode symbol | EL1 | | EL2 | | EL3 | |
| | Es/mV | Estd/mV | Es/mV | Estd/mV | Es/mV | Estd/mV |
| HCO$_3^-$ selectivity coefficient calculating solution | 47.07 | 146.02 | 45.93 | 144.50 | 43.35 | 143.88 |
| K$_s$ (Cl$^-$, HCO$_3^-$) | 0.10 | | 0.56 | | 0.87 | |

(2) Calculation of acetate ion (AcO$^-$) selectivity coefficient

In a similar manner to the above (1), an acetate ion (AcO$^-$) selectivity coefficient calculating solution was prepared.

Sodium acetate (NaOAc) and sodium chloride (NaCl) were dissolved in a HEPES buffer (100 mM, pH 8.0), to give a 10 mM sodium acetate (NaOAc) and 100 mM sodium chloride (NaCl)/HEPES buffer, and the 10 mM sodium acetate (NaOAc) and 100 mM sodium chloride (NaCl)/HEPES buffer was used as the acetate ion (AcO$^-$) selectivity coefficient calculating solution.

In a vial, 2 ml of the acetate ion (AcO$^-$) selectivity coefficient calculating solution was put, the vial was set in an electrolyte analyzer ELA08 manufactured by A & T Co., Ltd., and measurement was performed at room temperature. The measurement was performed by first measuring a potential difference $E_{Std}$ of the standard solution for one calibration curve solution using the ion-selective electrode (the electrode 1, 2, or 3) and the reference electrode connected to each other via the potentiometer, and then measuring a potential difference $E_S$ of the calibration curve solution.

Here, by substituting various parameters into the above formulas (19) and (8B) in a similar manner to the above "(1) Calculation of bicarbonate Ion (HCO$_3^-$) selectivity coefficient" except that the above potential difference $E_S$ is used as the potential difference $E_S$, and concentrations of a chloride ion (Cl$^-$) and an acetate ion (AcO$^-$) in the acetate ion (AcO$^-$) selectivity coefficient calculating solution are used as C(B1) and C(B2), respectively, a selectivity coefficient (Ks(Cl$^-$, AcO$^-$)) for the acetate ion (AcO$^-$) when the chloride ion (Cl$^-$) is used as an ion to be measured is obtained.

Table 5 presents the potential difference Es, the potential difference $E_{Std}$ of the standard solution, and the selectivity coefficient (Ks(Cl$^-$, AcO$^-$)) when the acetate ion (AcO$^-$) selectivity coefficient calculating solution is measured with each of the electrodes EL1, EL2, and EL3.

[Table 5]

| Table 5 Measured values of potential difference $E_S$ and potential difference $E_{Std}$ of standard solution, and acetate ion (AcO$^-$) selectivity coefficient when electrode EL1, EL2, or EL3 is used as electrode S | | | | | | |
|---|---|---|---|---|---|---|
| Electrode symbol | EL1 | | EL2 | | EL3 | |
| | Es/mV | Estd/mV | Es/mV | Estd/mV | Es/mV | Estd/mV |
| AcO$^-$ selectivity coefficient calculating solution | 46.66 | 145.55 | 44.78 | 143.75 | 44.30 | 143.72 |
| K$_s$ (Cl$^-$, AcO$^-$) | 0.07 | | 0.76 | | 0.32 | |

(Creation of matrix A)

A matrix A was created from the values of the selectivity coefficients obtained in the above "Calculation of selectivity coefficient" for the electrodes EL1, EL2, and EL3.

The following Table 6 collectively presents the selectivity coefficients obtained in the above "Calculation of selectivity coefficient". Here, the selectivity coefficient (Ks(Cl⁻, Cl⁻)) for an ion to be measured (chloride ion (Cl⁻)) itself is set to 1.

[Table 6]

| Table 6 Selectivity coefficient of each ion-selective electrode for ion to be measured | | | | |
|---|---|---|---|---|
| Electrode symbol | | EL1 | EL2 | EL3 |
| Selectivity coefficient | $K_s$ (Cl⁻, Cl⁻) | 1 | 1 | 1 |
| | $K_s$ (Cl⁻, HCO$_3$⁻) | 0.10 | 0.56 | 0.87 |
| | $K_s$ (Cl⁻, AcO⁻) | 0.07 | 0.76 | 0.32 |

A matrix A corresponding to the selectivity coefficients presented in Table 6 above can be expressed as follows. Here, for determining the following matrix A, the electrodes EL1, EL2, and EL3 were defined as a first ion-selective electrode (ion-selective electrode S1), a second ion-selective electrode (ion-selective electrode S2), and a third ion-selective electrode (ion-selective electrode S3), respectively. In the following matrix A, selectivity coefficients $K_s$(Cl⁻, Cl⁻), $K_s$(Cl⁻, HCO$_3$⁻), and $K_s$(Cl⁻, AcO⁻) for an i-th (i is 1 or more and 3 or less) ion-selective electrode among these three ion-selective electrodes are represented by $K_{Si}$(Cl⁻, Cl⁻), $K_{Si}$(Cl⁻, HCO$_3$⁻), and $K_{Si}$(Cl⁻, AcO⁻), respectively.

[Mathematical formula 32]

$$\text{Matrix A} = \begin{pmatrix} K_{S1}(Cl^-,Cl^-) & K_{S1}(Cl^-,HCO_3^-) & K_{S1}(Cl^-,AcO^-) \\ K_{S2}(Cl^-,Cl^-) & K_{S2}(Cl^-,HCO_3^-) & K_{S2}(Cl^-,AcO^-) \\ K_{S3}(Cl^-,Cl^-) & K_{S3}(Cl^-,HCO_3^-) & K_{S3}(Cl^-,AcO^-) \end{pmatrix} = \begin{pmatrix} 1 & 0.10 & 0.07 \\ 1 & 0.56 & 0.76 \\ 1 & 0.87 & 0.32 \end{pmatrix}$$

This matrix A was input to a cell (A1:C3) of spreadsheet software Excel, and an absolute value ‖A‖ of the determinant was determined using a function of "= MDETERM (A1:C3)" and found to be 0.42.

Here, the calculation of the absolute value ‖A‖ in Excel can be specifically performed as follows. First, 1 is input to a cell A1, 1 is input to a cell A2, 1 is input to a cell A3, 0.10 is input to a cell B1, 0.56 is input to a cell B2, 0.87 is input to a cell B3, 0.07 is input to a cell C1, 0.76 is input to a cell C2, and 0.32 is input to a cell C3. Next, when "= MDETERM (A1:C3)" is input to any cell other than these cells, a calculated value of "= MDETERM (A1:C3)" can be obtained as a value of the determinant |A| (in this case, -0.42). Then, by applying an ABS function to the value of the determinant |A|, an absolute value of the determinant |A| can be obtained, and this value can be adopted as the absolute value ‖A‖.

Here, in the above Tables 4 to 6 and the above determinant A, the values of $K_s$(Cl⁻, HCO$_3$⁻) and $K_s$(Cl⁻, AcO⁻) for each ion-selective electrode are shown in a form of being rounded off to the third decimal place for convenience of documentation. Note that, in calculation of an inverse matrix A⁻¹ and calculation of a concentration of each ion, which will be described later in the following "1-8 Calculation of ion concentration of sample solution", values were used in a manner that their third decimal places and subsequent decimal places were taken into consideration, instead of adopting values obtained by rounding off these values to the third decimal place.

1-7 Measurement of potential difference Es of sample solution

In a vial, 2 ml of a sample solution X was put, the vial was set in an electrolyte analyzer ELA08 of A & T Co., Ltd., and measurement was performed at room temperature. The measurement was performed by first measuring a potential difference $E_{Std}$ of the standard solution using the ion-selective electrode (the electrode EL1, the electrode EL2, or the electrode EL3) and the reference electrode connected to each other via the potentiometer, and then measuring a potential difference Es of the sample solution X. Table 7 presents measured values and calculated values obtained from the measured values (calculated values obtained with the electrode S). Correction was performed with $E_{std}$ in Table 7 using the values of Slope in Table 4, determined in the above "1-5 Creation of calibration curve (calculation of values of $E_{std}$ and Slope)".

[Table 7]

| Table 7 Measured values of potential difference $E_S$ and potential difference $E_{Std}$ of standard solution, and calculated value obtained with electrode S when electrode EL1, EL2, or EL3 is used as electrode S | | | |
|---|---|---|---|
| Electrode symbol | Es/mV | Estd/mV | Calculated value obtained with electrode S |
| EL1 | 45.68 | 146.21 | 108.72 |
| EL2 | 42.37 | 144.49 | 125.10 |
| EL3 | 39.85 | 144.26 | 130.24 |

[Mathematical formula 33]

$$\text{Calculated value obtained with electrode S} = 10^{\left(\dfrac{E_S - E_{0,S}}{\text{Slope}}\right)}$$

$$= 10^{\left(\dfrac{E_S - E_{Std}}{\text{Slope}} + \log_{10} C_{Std}\right)} \quad (20)$$

1-8 Calculation of ion concentration of sample solution

An ion concentration of the sample solution (sample solution X) was calculated as follows.

First, an inverse matrix $A^{-1}$ of the matrix A obtained in the above "1-6 Calculation of selectivity coefficient and creation of matrix A for ion-selective electrode" is determined. This matrix A is obtained by using the electrodes EL1, EL2, and EL3 as the ion-selective electrode S1, the ion-selective electrode S2, and the ion-selective electrode S3, respectively. The inverse matrix $A^{-1}$ was determined by inputting the matrix A to a cell (A1:C3) of spreadsheet software Excel and using a function of "= MINVERSE (A1:C3)".

Here, the calculation of the inverse matrix $A^{-1}$ in Excel can be performed by inputting "= MINVERSE (A1:C3)" to any cell away from the cells A1 to C3 input in the above "1-6 Calculation of selectivity coefficient and creation of matrix A for ion-selective electrode", and a group of calculated values displayed in the cell input as "= MINVERSE (A1:C3)" and its neighboring cells can be adopted as the inverse matrix $A^{-1}$.

[Mathematical formula 34]

$$A^{-1} = \begin{pmatrix} 1.16 & -0.07 & -0.09 \\ -1.06 & -0.60 & 1.66 \\ -0.74 & 1.85 & -1.10 \end{pmatrix}$$

By applying the inverse matrix $A^{-1}$ to the "calculated value obtained with electrode S" described in Table 7, obtained in the above "1-7 Measurement of potential difference $E_S$ of sample solution", a concentration $C(Cl^-)$ of a chloride ion ($Cl^-$), a concentration $C(HCO_3^-)$ of a bicarbonate ion ($HCO_3^-$), and a concentration $C(AcO^-)$ of an acetate ion ($AcO^-$) were determined. Here, the "calculated value obtained with electrode S" for the electrode EL1 was used as "calculated value obtained with electrode S1", the "calculated value obtained with electrode S" for the electrode EL2 was used as "calculated value obtained with electrode S2", and the "calculated value obtained with electrode S" for the electrode EL3 was used as "calculated value obtained with electrode S3". As a result, $C(Cl^-)$ = 105.7 mM, $C(HCO_3^-)$ = 26.0 mM, and $C(AcO^-)$ = 6.09 mM.

[Mathematical formula 35]

$$\begin{pmatrix} C(Cl^-) \\ C(HCO_3^-) \\ C(AcO^-) \end{pmatrix} = A^{-1} \cdot \begin{pmatrix} \text{Calculated value obtained with electrode S1} \\ \text{Calculated value obtained with electrode S2} \\ \text{Calculated value obtained with electrode S3} \end{pmatrix}$$

$$= \begin{pmatrix} 1.16 & -0.07 & -0.09 \\ -1.06 & -0.60 & 1.66 \\ -0.74 & 1.85 & -1.10 \end{pmatrix} \cdot \begin{pmatrix} \text{Calculated value obtained with electrode S1} \\ \text{Calculated value obtained with electrode S2} \\ \text{Calculated value obtained with electrode S3} \end{pmatrix}$$

Here, the inverse matrix $A^{-1}$, the values of $K_s(Cl^-, HCO_3^-)$ and $K_S(Cl^-, AcO^-)$ for each ion-selective electrode determined in the above "1-6 Calculation of selectivity coefficient and creation of matrix A for ion-selective electrode", and the value of" calculated value obtained with electrode S" obtained in the above "1-7 Measurement of potential difference $E_S$ of sample solution" are shown in a form of being rounded off to the third decimal place for convenience of documentation. Note that, in calculation of a concentration of each ion, values were used in a manner that their third decimal places and subsequent decimal places were taken into consideration, instead of adopting values obtained by rounding off these values to the third decimal place. Then, the processing of rounding off a numerical value was not performed in the middle of calculating the concentration of each ion, but was performed only for a finally obtained concentration value.

A concentration of each ion in the following Example 2 and Comparative Example 1 was calculated similarly.

Note that, due to an error caused by such processing of rounding off a numerical value, a calculation result for a concentration of each ion determined in the present Example and the following Example 2 and Comparative Example 1 may have a slight deviation from a concentration value determined by using, as values of "calculated value obtained with electrode S", values rounded off to the third decimal place and using, as an inverse matrix $A^{-1}$, an inverse matrix including, as a value of each element, values rounded off to the third decimal place, and simply combining them.

The operations of the above "1-7 Measurement of potential difference $E_S$ of sample solution" and the above "1-8 Calculation of ion concentration of sample solution" were repeated six times, and a relative standard deviation (percentage of a value obtained by dividing a standard deviation by an average value) (%) was determined for each of a chloride ion, a bicarbonate ion, and an acetate ion.

Table 18 presents the results.

[Example 2]

2-1 Configuration of ion concentration measurement device

As an ion concentration measurement device, a device having the configuration described in the above "Configuration of ion concentration measurement device" was used as in Example 1. Here, as an ion-selective electrode, a reference electrode, a potentiometer, a tank to contain a solution, and an arithmetic device, those described below were used.

(Ion-selective electrode)

As ion-selective electrodes, the ion-selective electrode of the electrode symbol EL1 ("electrode EL1"), the ion-selective electrode of the electrode symbol EL2 ("electrode EL2"), and the ion-selective electrode of the electrode symbol EL4 ("electrode EL4") obtained in the above "Preparation of ion-selective electrode" were used.

(Reference electrode, potentiometer, arithmetic device, and tank to contain a solution)

As a reference electrode, a potentiometer, an arithmetic device, and a tank to contain a solution, those described in the above "Configuration of ion concentration measurement device" were used as in Example 1.

2-2 Sample solution

A sample solution same as the sample solution X used in Example 1 was used.

2-3 Standard solution

A standard solution same as that in Example 1 was used.

2-4 Calibration curve solution

A calibration curve solution same as that in Example 1 was used.

2-5 Creation of calibration curve (calculation of values of $E_{std}$ and Slope)

Creation of a calibration curve and calculation of a value of Slope were performed in the same manner as in Example 1 except that the electrode EL1, the electrode EL2, or the electrode EL4 was used as the ion-selective electrode. Table 8 presents a determined value of Slope.

[Table 8]

| Table 8 Potential and Slope value obtained by measuring calibration curve solution using electrode EL1, EL2, or EL4 as electrode S | | | | | | |
|---|---|---|---|---|---|---|
| Electrode symbol | | EL1 | | EL2 | | EL4 |
| Calibration curve solution | | Es/mV | Estd/mV | Es/mV | Estd/mV | Es/mV | Estd/mV |
| Cl- | 80 mM | 52.40 | 146.36 | 51.93 | 144.63 | 37.39 | 144.44 |
| Cl- | 100 mM | 47.54 | 146.28 | 47.14 | 144.56 | 31.84 | 144.34 |
| Cl- | 120 mM | 43.66 | 146.31 | 43.31 | 144.59 | 27.43 | 144.38 |
| Slope/(mV/dec) | | -49.37 | | -48.71 | | -56.25 |

2-6 Calculation of selectivity coefficient and creation of matrix A for ion-selective electrode

(Calculation of selectivity coefficient)

For each of the electrodes (electrodes EL1, EL2, and EL4) of the electrode symbols EL1, EL2, and EL4, a selectivity coefficient for an ion to be measured was determined. The calculation of the selectivity coefficient was performed in the same manner as in Example 1. Tables 9 and 10 present the measured values and the determined selectivity coefficient values.

[Table 9]

| Table 9 Measured values of potential difference $E_S$ and potential difference $E_{Std}$ of standard solution, and bicarbonate ion ($HCO_3^-$) selectivity coefficient of electrode S when electrode EL1, EL2, or EL4 is used as electrode S | | | | | | |
|---|---|---|---|---|---|---|
| Electrode symbol | EL1 | | EL2 | | EL4 | |
| | Es/mV | Estd/mV | Es/mV | Estd/mV | Es/mV | Estd/mV |
| $HCO_3^-$ selectivity coefficient calculating solution | 47.16 | 146.11 | 45.97 | 144.54 | 29.77 | 143.31 |
| $K_s$ (Cl-, $HCO_3^-$) | 0.10 | | 0.56 | | 0.44 | |

[Table 10]

| Table 10 Measured values of potential difference Es and potential difference E$_{Std}$ of standard solution, and acetate ion (AcO$^-$) selectivity coefficient of electrode S when electrode EL1, EL2, or EL4 is used as electrode S | | | | | | |
|---|---|---|---|---|---|---|
| Electrode symbol | | EL1 | | EL2 | | EL4 |
| | Es/mV | Estd/mV | Es/mV | Estd/mV | Es/mV | Estd/mV |
| AcO$^-$ selectivity coefficient calculating solution | 46.66 | 145.55 | 44.78 | 143.75 | 30.88 | 143.72 |
| K$_s$ (Cl$^-$, AcO$^-$) | 0.07 | | 0.76 | | 0.14 | |

(Creation of matrix A)

A matrix A was created from the values of the selectivity coefficients obtained in the above "Calculation of selectivity coefficient" for the electrodes EL1, EL2, and EL4.

The following Table 11 collectively presents the selectivity coefficients obtained in the above "Calculation of selectivity coefficient". Here, the selectivity coefficient (Ks(Cl$^-$, Cl$^-$)) for an ion to be measured (chloride ion (Cl$^-$)) itself is set to 1.

[Table 11]

| Table 11 Selectivity coefficient of each ion-selective electrode for ion to be measured | | | | |
|---|---|---|---|---|
| Electrode symbol | | EL1 | EL2 | EL4 |
| Selectivity coefficient | K$_s$ (Cl$^-$, Cl$^-$) | 1 | 1 | 1 |
| | K$_s$ (Cl$^-$, HCO$_3^-$) | 0.10 | 0.56 | 0.44 |
| | K$_s$ (Cl$^-$, AcO$^-$) | 0.07 | 0.76 | 0.14 |

A matrix A corresponding to the selectivity coefficients presented in Table 11 above can be expressed as follows. Here, for determining the following matrix A, the electrodes EL1, EL2, and EL4 were defined as a first ion-selective electrode (ion-selective electrode S1), a second ion-selective electrode (ion-selective electrode S2), and a third ion-selective electrode (ion-selective electrode S3), respectively. In the following matrix A, selectivity coefficients K$_s$(Cl$^-$, Cl$^-$), K$_s$(Cl$^-$, HCO$_3^-$), and K$_s$(Cl$^-$, AcO$^-$) for an i-th (i is 1 or more and 3 or less) ion-selective electrode among these three ion-selective electrodes are represented by K$_{Si}$(Cl$^-$, Cl$^-$), K$_{Si}$(Cl$^-$, HCO$_3^-$), and K$_{Si}$(Cl$^-$, AcO$^-$), respectively.

[Mathematical formula 36]

$$
\text{Matrix A} = \begin{pmatrix} K_{S1}(Cl^-,Cl^-) & K_{S1}(Cl^-,HCO_3^-) & K_{S1}(Cl^-,AcO^-) \\ K_{S2}(Cl^-,Cl^-) & K_{S2}(Cl^-,HCO_3^-) & K_{S2}(Cl^-,AcO^-) \\ K_{S3}(Cl^-,Cl^-) & K_{S3}(Cl^-,HCO_3^-) & K_{S3}(Cl^-,AcO^-) \end{pmatrix} = \begin{pmatrix} 1 & 0.10 & 0.07 \\ 1 & 0.56 & 0.76 \\ 1 & 0.44 & 0.14 \end{pmatrix}
$$

This matrix A was input to a cell (A1:C3) of spreadsheet software Excel, and an absolute value ‖A‖ of the determinant was determined using a function of "= MDETERM (A1:C3)" and found to be 0.20.

2-7 Measurement of potential difference E$_S$ of sample solution

Measurement of a potential difference E$_S$ of a sample solution was performed in the same manner as in Example 1 except that the electrode EL1, the electrode EL2, or the electrode EL4 was used as the ion-selective electrode. Table 12 presents measured values and calculated values obtained from the measured values (calculated values obtained with the electrode S). Correction was performed with E$_{std}$ in Table 12 using the values of Slope in Table 8, determined in the same manner as in Example 1.

[Table 12]

Table 12 Measured values of potential difference $E_S$ and potential difference $E_{Std}$ of standard solution, and calculated value obtained with electrode S when electrode EL1, EL2, or EL4 is used as electrode S

| Electrode symbol | Es/mV | Estd/mV | Calculated value obtained with electrode S |
|---|---|---|---|
| EL1 | 44.54 | 145.14 | 109.02 |
| EL2 | 41.35 | 143.49 | 125.01 |
| EL4 | 26.57 | 143.22 | 118.51 |

2-8 Calculation of ion concentration of sample solution

An ion concentration of the sample solution was calculated in the same manner as in Example 1.

First, an inverse matrix $A^{-1}$ of the matrix A obtained in the above "2-6 Calculation of selectivity coefficient and creation of matrix A for ion-selective electrode" is determined in the same manner as in Example 1. This matrix A is obtained by using the electrodes EL1, EL2, and EL4 as the ion-selective electrode S1, the ion-selective electrode S2, and the ion-selective electrode S3, respectively. The inverse matrix $A^{-1}$ was determined by inputting the matrix A to a cell (A1:C3) of spreadsheet software Excel and using a function of "= MINVERSE (A1:C3)".

[Mathematical formula 37]

$$A^{-1} = \begin{pmatrix} 1.27 & -0.09 & -0.18 \\ -3.09 & -0.32 & 3.41 \\ 0.61 & 1.66 & -2.27 \end{pmatrix}$$

By applying the inverse matrix $A^{-1}$ to the "calculated value obtained with electrode S" described in Table 12, obtained in the above "2-7 Measurement of potential difference Es of sample solution", a concentration $C(Cl^-)$ of a chloride ion ($Cl^-$), a concentration $C(HCO_3^-)$ of a bicarbonate ion ($HCO_3^-$), and a concentration $C(AcO^-)$ of an acetate ion ($AcO^-$) were determined. Here, the "calculated value obtained with electrode S" for the electrode EL1 was used as "calculated value obtained with electrode S1", the "calculated value obtained with electrode S" for the electrode EL2 was used as "calculated value obtained with electrode S2", and the "calculated value obtained with electrode S" for the electrode EL4 was used as "calculated value obtained with electrode S3". As a result, $C(Cl^-)$ = 105.7 mM, $C(HCO_3^-)$ = 27.6 mM, and $C(AcO^-)$ = 5.21 mM.

[Mathematical formula 38]

$$\begin{pmatrix} C(Cl^-) \\ C(HCO_3^-) \\ C(AcO^-) \end{pmatrix} = A^{-1} \cdot \begin{pmatrix} \text{Calculated value obtained with electrode S1} \\ \text{Calculated value obtained with electrode S2} \\ \text{Calculated value obtained with electrode S3} \end{pmatrix}$$

$$= \begin{pmatrix} 1.27 & -0.09 & -0.18 \\ -3.09 & -0.32 & 3.41 \\ 0.61 & 1.66 & -2.27 \end{pmatrix} \cdot \begin{pmatrix} \text{Calculated value obtained with electrode S1} \\ \text{Calculated value obtained with electrode S2} \\ \text{Calculated value obtained with electrode S3} \end{pmatrix}$$

The operations of the above "2-7 Measurement of potential difference Es of sample solution" and the above "2-8 Calculation of ion concentration of sample solution" were repeated six times, and a relative standard deviation (%) was determined in the same manner as in Example 1. Table 18 presents the results.

[Comparative Example 1]

C1-1 Configuration of ion concentration measurement device

As an ion concentration measurement device, a device having the configuration described in the above "Configuration of ion concentration measurement device" was used as in Example 1. Here, as an ion-selective electrode, a reference electrode, a potentiometer, a tank to contain a solution, and an arithmetic device, those described below were used.

(Ion-selective electrode)

As ion-selective electrodes, the ion-selective electrode of the electrode symbol EL5 ("electrode EL5"), the ion-selective electrode of the electrode symbol EL6 ("electrode EL6"), and the ion-selective electrode of the electrode symbol EL7 ("electrode EL7") obtained in the above "Preparation of ion-selective electrode" were used.

(Reference electrode, potentiometer, arithmetic device, and tank to contain a solution)

As a reference electrode, a potentiometer, an arithmetic device, and a tank to contain a solution, those described in the above "Configuration of ion concentration measurement device" were used as in Example 1.

C1-2 Sample solution

A sample solution same as the sample solution X used in Example 1 was used.

C1-3 Standard solution

A standard solution same as that in Example 1 was used.

C1-4 Calibration curve solution

A standard solution same as that in Example 1 was used.

C1-5 Creation of calibration curve (calculation of values of $E_{std}$ and Slope)

Creation of a calibration curve and calculation of a value of Slope were performed in the same manner as in Example 1 except that the electrode EL5, the electrode EL6, or the electrode EL7 was used as the ion-selective electrode. Table 13 presents a determined value of Slope.

[Table 13]

| Table 13 Potential and Slope value obtained by measuring calibration curve solution using electrode EL5, EL6, or EL7 as electrode S | | | | | | |
|---|---|---|---|---|---|---|
| Electrode symbol | | EL5 | | EL6 | | EL7 |
| Calibration curve solution | Es/mV | Estd/mV | Es/mV | Estd/mV | Es/mV | Estd/mV |
| Cl-　　　　80 mM | 71.04 | 145.47 | 71.27 | 143.80 | 70.67 | 143.54 |
| Cl-　　　　100 mM | 66.96 | 145.18 | 67.30 | 143.52 | 66.66 | 143.24 |
| Cl-　　　　120 mM | 63.86 | 145.18 | 64.27 | 143.51 | 63.63 | 143.24 |
| Slope/(mV/dec) | -39.11 | | -38.11 | | -38.29 | |

C1-6 Calculation of selectivity coefficient and creation of matrix A for ion-selective electrode

(Calculation of selectivity coefficient)

For each of the electrodes (electrodes EL5, EL6, and EL7) of the electrode symbols EL5, EL6, and EL7, a selectivity coefficient for an ion to be measured was determined. The calculation of the selectivity coefficient was performed in the same manner as in Example 1. Tables 14 and 15 present the measured values and the determined selectivity coefficient values.

[Table 14]

| Table 14 Measured values of potential difference $E_S$ and potential difference $E_{Std}$ of standard solution, and bicarbonate ion ($HCO_3^-$) selectivity coefficient when electrode EL5, EL6, or EL7 is used as electrode S | | | | | | |
|---|---|---|---|---|---|---|
| Electrode symbol | EL5 | | EL6 | | EL7 | |
| | Es/mV | Estd/mV | Es/mV | Estd/mV | Es/mV | Estd/mV |
| $HCO_3^-$ selectivity coefficient | 66.05 | 145.03 | 65.99 | 143.48 | 65.80 | 143.11 |
| $K_s$ ($Cl^-$, $HCO_3^-$) | 0.46 | | 0.80 | | 0.45 | |

[Table 15]

| Table 15 Measured values of potential difference Es and potential difference $E_{Std}$ of standard solution, and acetate ion ($AcO^-$) selectivity coefficient of electrode S when electrode EL5, EL6, or EL7 is used as electrode S | | | | | | |
|---|---|---|---|---|---|---|
| Electrode symbol | EL5 | | EL6 | | EL7 | |
| | Es/mV | Estd/mV | Es/mV | Estd/mV | Es/mV | Estd/mV |
| $AcO^-$ | 66.16 | 145.05 | 65.97 | 143.33 | 66.44 | 143.58 |
| selectivity coefficient | | | | | | |
| $K_s$ ($Cl^-$, $AcO^-$) | 0.40 | | 0.71 | | 0.34 | |

(Creation of matrix A)

A matrix A was created from the values of the selectivity coefficients obtained in the above "Calculation of selectivity coefficient" for the electrodes EL5, EL6, and EL7.

The following Table 16 collectively presents the selectivity coefficients obtained in the above "Calculation of selectivity coefficient". Here, the selectivity coefficient (Ks($Cl^-$, $Cl^-$)) for an ion to be measured (chloride ion ($Cl^-$)) itself is set to 1.

[Table 16]

| Table 16 Selectivity coefficient of each ion-selective electrode for ion to be measured | | | | |
|---|---|---|---|---|
| Electrode symbol | | EL5 | EL6 | EL7 |
| Selectivity coefficient | $K_s$ ($Cl^-$, $Cl^-$) | 1 | 1 | 1 |
| | $K_s$ ($Cl^-$, $HCO_3^-$) | 0.46 | 0.80 | 0.45 |
| | $K_s$ ($Cl^-$, $AcO^-$) | 0.40 | 0.71 | 0.34 |

A matrix A corresponding to the selectivity coefficients presented in Table 16 above can be expressed as follows.

Here, for determining the following matrix A, the electrodes EL5, EL6, and EL7 were defined as a first ion-selective electrode (ion-selective electrode S1), a second ion-selective electrode (ion-selective electrode S2), and a third ion-selective electrode (ion-selective electrode S3), respectively. In the following matrix A, selectivity coefficients $K_S$($Cl^-$, $Cl^-$), $K_S$($Cl^-$, $HCO_3^-$), and $K_S$($Cl^-$, $AcO^-$) for an i-th (i is 1 or more and 3 or less) ion-selective electrode among these three ion-selective electrodes are represented by $K_{Si}$($Cl^-$, $Cl^-$), $K_{Si}$($Cl^-$, $HCO_3^-$), and $K_{Si}$($Cl^-$, $AcO^-$), respectively.

[Mathematical formula 39]

$$\text{Matrix A} = \begin{pmatrix} K_{S1}(Cl^-,Cl^-) & K_{S1}(Cl^-,HCO_3^-) & K_{S1}(Cl^-,AcO^-) \\ K_{S2}(Cl^-,Cl^-) & K_{S2}(Cl^-,HCO_3^-) & K_{S2}(Cl^-,AcO^-) \\ K_{S3}(Cl^-,Cl^-) & K_{S3}(Cl^-,HCO_3^-) & K_{S3}(Cl^-,AcO^-) \end{pmatrix} = \begin{pmatrix} 1 & 0.46 & 0.40 \\ 1 & 0.80 & 0.71 \\ 1 & 0.45 & 0.34 \end{pmatrix}$$

This matrix A was input to a cell (A1:C3) of spreadsheet software Excel, and an absolute value $\|A\|$ of the determinant was determined using a function of "= MDETERM (A1:C3)" and found to be 0.02.

C1-7 Measurement of potential difference $E_S$ of sample solution

Measurement of a potential difference $E_S$ of a sample solution was performed in the same manner as in Example 1 except that the electrode EL5, the electrode EL6, or the electrode EL7 was used as the ion-selective electrode. Table 17 presents measured values and calculated values obtained from the measured values (calculated values obtained with the electrode S). Correction was performed with $E_{std}$ in Table 17 using the values of Slope in Table 13, determined in the same manner as in Example 1.

[Table 17]

| Table 17 Measured values of potential difference $E_S$ and potential difference $E_{Std}$ of standard solution, and calculated value obtained with electrode S when electrode EL5, EL6, or EL7 is used as electrode S | | | |
|---|---|---|---|
| Electrode symbol | Es/mV | Estd/mV | Calculated value obtained with electrode S |
| EL5 | 62.93 | 144.36 | 120.82 |
| EL6 | 60.63 | 141.75 | 134.44 |
| EL7 | 62.26 | 141.96 | 120.61 |

C1-8 Calculation of ion concentration of sample solution

An ion concentration of the sample solution was calculated in the same manner as in Example 1.
First, an inverse matrix $A^{-1}$ of the matrix A obtained in the above "C1-6 Calculation of selectivity coefficient and creation of matrix A for ion-selective electrode" is determined in the same manner as in Example 1. This matrix A is obtained by using the electrodes EL5, EL6, and EL7 as the ion-selective electrode S1, the ion-selective electrode S2, and the ion-selective electrode S3, respectively. The inverse matrix $A^{-1}$ was determined by inputting the matrix A to a cell (A1:C3) of spreadsheet software Excel and using a function of "= MINVERSE (A1:C3)".

[Mathematical formula 40]

$$A^{-1} = \begin{pmatrix} 2.73 & -1.36 & -0.37 \\ -20.62 & 3.45 & 17.18 \\ 19.40 & -0.55 & -18.84 \end{pmatrix}$$

By applying the inverse matrix $A^{-1}$ to the "calculated value obtained with electrode S" described in Table 17, obtained in the above "C1-7 Measurement of potential difference $E_S$ of sample solution", a concentration $C(Cl^-)$ of a chloride ion $(Cl^-)$, a concentration $C(HCO_3^-)$ of a bicarbonate ion $(HCO_3^-)$, and a concentration $C(AcO^-)$ of an acetate ion $(AcO^-)$ were determined. Here, the "calculated value obtained with electrode S" for the electrode EL5 was used as "calculated value obtained with electrode S1", the "calculated value obtained with electrode S" for the electrode EL6 was used as "calculated value obtained with electrode S2", and the "calculated value obtained with electrode S" for the electrode EL7 was used as "calculated value obtained with electrode S3". As a result, $C(Cl^-)$ = 102.3 mM, $C(HCO_3^-)$ = 43.2 mM, and $C(AcO^-)$ = -3.46 mM.

[Mathematical formula 41]

$$
\begin{pmatrix} C(Cl^-) \\ C(HCO_3^-) \\ C(AcO^-) \end{pmatrix} = A^{-1} \cdot \begin{pmatrix} \text{Calculated value obtained with electrode S1} \\ \text{Calculated value obtained with electrode S2} \\ \text{Calculated value obtained with electrode S3} \end{pmatrix}
$$

$$
= \begin{pmatrix} 2.73 & -1.36 & -0.37 \\ -20.62 & 3.45 & 17.18 \\ 19.40 & -0.55 & -18.84 \end{pmatrix} \cdot \begin{pmatrix} \text{Calculated value obtained with electrode S1} \\ \text{Calculated value obtained with electrode S2} \\ \text{Calculated value obtained with electrode S3} \end{pmatrix}
$$

The operations of the above "C1-7 Measurement of potential difference Es of sample solution" and the above "C1-8 Calculation of ion concentration of sample solution" were repeated six times, and a relative standard deviation (%) was determined in the same manner as in Example 1. Table 18 presents the results.

[Table 18]

| Table 18 Summary of results | | | | | |
|---|---|---|---|---|---|
| | | | Measurement results | | |
| Sample solution | | | Example 1 | Example 2 | Comparative Example 1 |
| Ion | Concentration/mM | ‖A‖ | 0.42 | 0.20 | 0.02 |
| Cl⁻ | 105 | Measurement average value/mM | 105.9 | 105.7 | 102.9 |
| | | Relative standard deviation/% | 0.1 | 0.2 | 0.4 |
| HCO₃⁻ | 24 | Measurement average value/mM | 25.6 | 27.6 | 39.4 |
| | | Relative standard deviation/% | 1.1 | 2.0 | 9.9 |
| AcO⁻ | 6 | Measurement average value/mM | 6.2 | 5.2 | -0.1 |
| | | Relative standard deviation/% | 3.6 | 5.9 | 100 or more |

Reference Signs List

1    Ion-selective electrode

2    Reference electrode

3    Potentiometer

4    Tank to contain a solution

5    Internal electrode

6    Internal liquid

7    Ion-selective membrane

8    Liquid junction

9    Sample solution

**Claims**

1. An ion concentration measurement device comprising

   two or more kinds of ion-selective electrodes that have mutually different selectivity coefficient ratios for a plurality of ions and satisfy the following formulas (1) and (2),
   wherein the two or more kinds of ion-selective electrodes are constituted by n kinds of ion-selective electrodes, and
   wherein n is the number of kinds of the plurality of ions and is an integer of 2 or more: [Mathematical formula 1]

$$\text{Matrix A} = \begin{pmatrix} K_{S1}(B1,B1) & K_{S1}(B1,B2) & \cdots & K_{S1}(B1,Bj) & \cdots & K_{S1}(B1,Bn) \\ K_{S2}(B1,B1) & K_{S2}(B1,B2) & \cdots & K_{S2}(B1,Bj) & \cdots & K_{S2}(B1,Bn) \\ \vdots & \vdots & & \vdots & & \vdots \\ K_{Si}(B1,B1) & K_{Si}(B1,B2) & \cdots & K_{Si}(B1,Bj) & \cdots & K_{Si}(B1,Bn) \\ \vdots & \vdots & & \vdots & & \vdots \\ K_{Sn}(B1,B1) & K_{Sn}(B1,B2) & \cdots & K_{Sn}(B1,Bj) & \cdots & K_{Sn}(B1,Bn) \end{pmatrix} \quad (1)$$

$$\|A\| > 0.1 \quad (2)$$

   in formula (1), each component of the matrix A represents an ion selectivity coefficient, wherein
   Bj represents a j-th ion among n kinds of ions constituting the plurality of ions,
   Si is a symbol representing an i-th ion-selective electrode constituting the n kinds of ion-selective electrodes,
   i and j are each independently an integer of 1 to n, and
   $K_{Si}(B1, Bj)$ represents an ion selectivity coefficient for the ion Bj when an ion B1 is used as a reference in the ion-selective electrode Si; and
   in formula (2), $\|A\|$ is an absolute value of a determinant of the matrix A.

2. The ion concentration measurement device according to claim 1, wherein an ion concentration is calculated by the following formula (3): [Mathematical formula 2]

$$\begin{pmatrix} C(B1) \\ C(B2) \\ \vdots \\ C(Bn) \end{pmatrix} = A^{-1} \cdot \begin{pmatrix} \text{Calculated value obtained with electrode S1} \\ \text{Calculated value obtained with electrode S2} \\ \vdots \\ \text{Calculated value obtained with electrode Sn} \end{pmatrix} \quad (3)$$

   in formula (3), $A^{-1}$ represents an inverse matrix of the matrix A, C(Bj) represents a concentration of the ion Bj (j is an integer of 1 to n), and a calculated value obtained with an electrode Si (i is an integer of 1 to n) indicates a value obtained by conversion of a potential difference measured with the ion-selective electrode Si according to the following formula: [Mathematical formula 3]

$$\text{Calculated value obtained with electrode Si} = 10^{\left(\frac{E_{Si} - E_{0,Si}}{Slope}\right)}$$

in the formula,

$E_{Si}$ is a potential difference generated between the ion-selective electrode Si and a reference electrode,

$E_{0,Si}$ is a standard potential difference generated between the ion-selective electrode Si and the reference electrode,

Slope is $2.303RT/aF$,

R is a gas constant,

T is an absolute temperature,

a is a valence of the ion B1, and

F is a Faraday constant.

3. An ion concentration measurement method comprising

a step of measuring an ion concentration in a sample solution using two or more kinds of ion-selective electrodes that have mutually different selectivity coefficient ratios for a plurality of ions and satisfy the following formulas (1) and (2),

wherein the two or more kinds of ion-selective electrodes are constituted by n kinds of ion-selective electrodes, and

wherein n is the number of kinds of the plurality of ions and is an integer of 2 or more: [Mathematical formula 4]

$$\text{Matrix } A = \begin{pmatrix} K_{S1}(B1,B1) & K_{S1}(B1,B2) & \cdots & K_{S1}(B1,Bj) & \cdots & K_{S1}(B1,Bn) \\ K_{S2}(B1,B1) & K_{S2}(B1,B2) & \cdots & K_{S2}(B1,Bj) & \cdots & K_{S2}(B1,Bn) \\ \vdots & \vdots & & \vdots & & \vdots \\ K_{Si}(B1,B1) & K_{Si}(B1,B2) & \cdots & K_{Si}(B1,Bj) & \cdots & K_{Si}(B1,Bn) \\ \vdots & \vdots & & \vdots & & \vdots \\ K_{Sn}(B1,B1) & K_{Sn}(B1,B2) & \cdots & K_{Sn}(B1,Bj) & \cdots & K_{Sn}(B1,Bn) \end{pmatrix} \quad (1)$$

$$\|A\| > 0.1 \quad (2)$$

in formula (1), each component of the matrix A represents an ion selectivity coefficient, wherein

Bj represents a j-th ion among n kinds of ions constituting the plurality of ions,

Si is a symbol representing an i-th ion-selective electrode constituting the n kinds of ion-selective electrodes,

i and j are each independently an integer of 1 to n, and

$K_{Si}(B1, Bj)$ represents an ion selectivity coefficient for the ion Bj when an ion B1 is used as a reference in the ion-selective electrode Si; and

in formula (2), $\|A\|$ is an absolute value of a determinant of the matrix A.

4. The ion concentration measurement method according to claim 3, wherein an ion concentration is calculated by the following formula (3): [Mathematical formula 5]

$$\begin{pmatrix} C(B1) \\ C(B2) \\ \vdots \\ C(Bn) \end{pmatrix} = A^{-1} \cdot \begin{pmatrix} \text{Calculated value obtained with electrode S1} \\ \text{Calculated value obtained with electrode S2} \\ \vdots \\ \text{Calculated value obtained with electrode Sn} \end{pmatrix} \qquad (3)$$

in formula (3), $A^{-1}$ represents an inverse matrix of the matrix A, $C(Bj)$ represents a concentration of the ion Bj (j is an integer of 1 to n), and a calculated value obtained with an electrode Si (i is an integer of 1 to n) indicates a value obtained by conversion of a potential difference measured with the ion-selective electrode Si according to the following formula:

[Mathematical formula 6]

$$\text{Calculated value obtained with electrode Si} = 10^{\left( \frac{E_{Si} - E_{0,Si}}{\text{Slope}} \right)}$$

in the formula,
$E_{Si}$ is a potential difference generated between the ion-selective electrode Si and a reference electrode,
$E_{0,Si}$ is a standard potential difference generated between the ion-selective electrode Si and the reference electrode,
Slope is 2.303RT/aF,
R is a gas constant,
T is an absolute temperature,
a is a valence of the ion B1, and
F is a Faraday constant.

[Fig. 1]

## INTERNATIONAL SEARCH REPORT

| | International application No. |
|---|---|
| | **PCT/JP2023/011705** |

**A. CLASSIFICATION OF SUBJECT MATTER**

**_G01N 27/416_**(2006.01)i
FI: G01N27/416 386G; G01N27/416 356

According to International Patent Classification (IPC) or to both national classification and IPC

**B. FIELDS SEARCHED**

Minimum documentation searched (classification system followed by classification symbols)

G01N27/416

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); Scopus

**C. DOCUMENTS CONSIDERED TO BE RELEVANT**

| Category* | Citation of document, with indication, where appropriate, of the relevant passages | Relevant to claim No. |
|---|---|---|
| X | JP 2000-121595 A (HITACHI, LTD.) 28 April 2000 (2000-04-28) paragraphs [0006], [0008], [0009], [0014]-[0017], [0037], [0038], tables 1, 3 | 1, 3 |
| Y | | 2, 4 |
| Y | GLAZIER, Scott A. et al. Evaluation of an overdetermined system based on multiple ion-selective electrodes of the same type. Talanta. 1988, vol. 35, no. 3, pages 215-219 page 215, right column, first paragraph, page 218, left column, second paragraph to right column, first paragraph | 2, 4 |
| A | OTTO, Matthias et al. Model Studies on Multiple Channel Analysis of Free Magnesium, Calcium, Sodium, and Potassium at Physiological Concentration Levels with Ion-Selective Electrodes. Anal. Chem. 1985, vol. 57, no. 13, pages 2647-2651, https://doi.org/10.1021/ac00290a049, ISSN: 0003-2700 page 2648, right column, fifth paragraph to page 2649, left column, first paragraph | 2, 4 |
| A | JP 7-167818 A (TOSHIBA CORP.) 04 July 1995 (1995-07-04) entire text, all drawings | 1, 3 |

☑ Further documents are listed in the continuation of Box C.     ☑ See patent family annex.

| * | Special categories of cited documents: | | |
|---|---|---|---|
| "A" | document defining the general state of the art which is not considered to be of particular relevance | "T" | later document published after the international filing date or priority date and not in conflict with the application but cited to understand the principle or theory underlying the invention |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 May 2023** | **23 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2023/011705**

**C.     DOCUMENTS CONSIDERED TO BE RELEVANT**

| Category* | Citation of document, with indication, where appropriate, of the relevant passages | Relevant to claim No. |
|---|---|---|
| A | JP 4-84750 A (SHIMADZU CORP.) 18 March 1992 (1992-03-18)<br>entire text, all drawings | 1, 3 |

Form PCT/ISA/210 (second sheet) (January 2015)

**INTERNATIONAL SEARCH REPORT**
Information on patent family members

International application No.

**PCT/JP2023/011705**

| Patent document cited in search report | | | Publication date (day/month/year) | Patent family member(s) | Publication date (day/month/year) |
|---|---|---|---|---|---|
| JP | 2000-121595 | A | 28 April 2000 | (Family: none) | |
| JP | 7-167818 | A | 04 July 1995 | US 5580441 A entire text, all drawings EP 667522 A2 | |
| JP | 4-84750 | A | 18 March 1992 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H1502360 A
- JP S54030094 A
- WO 2019163281 A
- JP H07167818 A

- JP H10318973 A
- JP H11132991 A
- JP 2003207476 A
- JP 2018004633 A

**Non-patent literature cited in the description**

- *CHEMICAL ABSTRACTS*, 80403-59-4
- *Anal. Chem.*, 1982, vol. 54 (7), 1224-1227

- *Pure Appl. Chem.*, 2000, vol. 72 (10), 1851-2082
- *Pure Appl. Chem.*, 2002, vol. 74 (6), 923-994